# EUROPEAN PATENT APPLICATION

(11) **EP 4 365 198 A1**
(43) Date of publication of application: **08.05.2024**
(21) Application number: 22831732.7
(22) Date of filing: 17.06.2022
(51) Int. Cl.: C07K 16/28, C12N 15/13, C12N 15/63, C07K 19/00, A61K 39/395, A61P 35/00, A61P 37/06

(54) **ANTI-CD3 ANTIBODY AND USE THEREOF**

(30) Priority: 02.07.2021 CN 202110752589
(71) Applicant: Ampsource Biopharma Shanghai Inc., Shanghai 201318 (CN)
(72) Inventor: WU, Cui, Shanghai 201318 (CN); GAO, Yongjuan, Shanghai 201318 (CN); ZHOU, Li, Shanghai 201318 (CN); DIAO, Jiasheng, Shanghai 201318 (CN); MA, Xinlu, Shanghai 201318 (CN); DONG, Zhao, Shanghai 201318 (CN); LI, Qiang, Shanghai 201318 (CN)
(74) Representative: HGF
(86) International application number: PCT/CN2022/099345
(87) International publication number: WO 2023/273914

(57) **Abstract**

Provided in the present invention is an antibody against human CD3 or an antigen-binding fragment thereof; also provided are a nucleic acid molecule encoding the antibody, an expression vector expressing the antibody and a host cell, as well as a method for producing the antibody. In addition, also provided in the present invention are a pharmaceutical composition comprising the antibody or the antigen-binding fragment thereof, a use thereof in the preparation of a drug, and a use of the drug as a drug for the prevention and/or treatment of various diseases, including tumors, organ transplantation and autoimmune diseases.

## Description

### TECHNICAL FIELD

The present application belongs to the field of therapeutic monoclonal antibodies and, in particular, to an antibody against human CD3 or an antigen-binding fragment thereof, and use of the antibody for anti-tumor, organ transplantation and autoimmune diseases.

### BACKGROUND

Immune cells, especially T cells, are at the core position throughout an immune response. Numerous CD molecules, such as CD3, CD4, CD8 and CD28, are present on the surface of the T cells, and the CD molecules widely participate in the whole process of immune effects of the T cells, such as antigen recognition, activation, proliferation or incapacity, apoptosis and the clearance of alloantigens or self-tolerance. Among these CD molecules, a CD3 molecule is particularly important. The CD3 molecule is an important differentiation antigen on the T cell membrane and is a characteristic marker of mature T cells. The CD3 molecule is composed of four different polypeptide chains, ε, γ, δ and ζ chains and forms a TCR-CD3 complex with a T-cell receptor (TCR) through a non-covalent bond. The CD3 molecule not only participates in the intracytoplasmic assembly of the TCR-CD3 complex but also transmits antigen stimulation signals through the immunoreceptor tyrosine-based activation motifs of the cytoplasmic regions of the polypeptide chains. The primary function of the CD3 molecule is to stabilize the structure of the TCR and transmit T cell activation signals. When the TCR specifically recognizes and binds to an antigen, CD3 participates in the transduction of a signal into the cytoplasm of the T cells as a first signal inducing T cell activation, which plays an extremely important role in the antigen recognition and immune response production process of the T cells.

Existing studies have shown that an antibody against human CD3 has the bidirectional functions of activating and inhibiting T cells. Thus, a monoclonal antibody may specifically bind to the CD3 molecule to activate or block the transduction of T cell activation signals, the efficacy of the monoclonal antibody has been well demonstrated in the acute anti-rejection shock therapy, treatment of graft-versus-host responses and prophylactic desensitization prior to organ transplantation, and the monoclonal antibody shows a broad application prospect in the anti-tumor, suppression of multiple organ transplant rejections and treatment of autoimmune diseases. It has been proved that the application of the anti-CD3 monoclonal antibody alone or in combination with an IL-1 receptor antagonist can prevent and reverse autoimmune diabetes in NOD mice and enhance immunomodulatory effects (Belghith M et al., Nat Med, 2003, 9(9): 1202-1208; Ablamunits V et al., Diabetes, 2012, 61(1): 145-154). During the clinical trial for treating subjects suffering from type 1 diabetes, the CD3 antibody also effectively improves insulin production and metabolic control, and patients suffering from type 1 diabetes, after being treated with an anti-CD3 monoclonal antibody Teplizumab, have significantly improved insulin levels and symptoms of metabolic abnormalities (Herold KC et al., N Engl J Med, 2002, 346(22): 1692-1698; Herold KC et al., Diabetes, 2005, 54(6): 1763-1769). The anti-CD3 antibody also reverses experimental allergic encephalomyelitis in Lewis rats and has an inhibitory effect on T helper type 1 (Th1)-mediated immunity (Tran G T et al., Intl Immunol, 2001, 13(9): 1109-1120. Additionally, the CD3 antibody is also widely used to mediate cellular functional bispecific antibodies. A bispecific antibody targeting CD3 can bind to T cell surface CD3 and tumor cell surface antigens, respectively, thereby reducing the distance between cytotoxic T cells (CTLs) and tumor cells and directly activating T cells to induce the direct killing of cancer cells by the T cells without relying on conventional dual activation signals of the T cells (Hipp S et al., Leukemia, 2017, 31(8): 1743-1751; Benonisson H et al., Molecular cancer therapeutics, 2019, 18(2): 312-322).

OKT3 having been launched earliest is a murine monoclonal antibody against human CD3 and manufactured by Ortho Pharmaceutical, USA. OKT3 has a good effect in preventing and treating immune rejections of organ transplantation and is approved for treating allograft rejection in kidney, liver and heart transplantation. However, the immunogenicity of OKT3, as a murine monoclonal antibody, results in the production of anti-OKT3 antibodies in more than 50% of patients, and these antibodies are mainly divided into two types: anti-individual antibody and anti-isotype antibody. The former blocks the binding of OKT3 to CD3 by neutralizing antigen binding sites, and the latter may cause severe side reactions in addition to accelerating the clearance of the monoclonal antibody. OKT3 may promote T cell over-activation to release a large amount of inflammatory factors such as interleukin-2 (IL-2), TNF-α, IFN-γ and interleukin-6 (IL-6), causing a variety of side effects, including fever, cold nausea, vomiting and headache, summarized to as "influenza-like", "cytokine release" or "first-dose syndrome". A small proportion of patients may experience more severe side effects such as cardiopulmonary distress, seizures, encephalopathy, meningitis, renal function insufficiency and graft thrombosis. Currently, a new generation of humanized anti-CD3 monoclonal antibodies, including otelixizumab, teplizumab and visilizumab, has greatly reduced affinity to an Fc receptor and significantly reduced side effects. However, the problems of T cell over-activation and cytokine secretion still exist. Therefore, during the development of an antibody targeting CD3, how to attenuate or avoid excessive cytokine storms is a primary concern, and the development of a CD3 antibody with higher specificity and better clinical efficacy is urgent and necessary, which will provide more medication options for patients with tumors, organ transplantation and autoimmune diseases.

### SUMMARY

The present application discloses an antibody that specifically binds to a CD3ε chain with high affinity or an antigen-binding fragment thereof. The present application further provides a nucleic acid molecule encoding the antibody or the antigen-binding fragment thereof, an expression vector, a host cell and a method for producing the antibody. The present application further provides a bispecific antibody, a multi-bispecific antibody and a pharmaceutical composition comprising the antibody or the antigen-binding fragment thereof. Additionally, the present application further provides use of an anti-CD3 antibody or an antigen-binding fragment thereof (alone or in combination with another active agent or therapy) disclosed in the present application for preparing a medicament for prevention and/or treatment of multiple diseases (comprising a tumor, organ transplantation and an autoimmune disease).

In a first aspect of the present application, the present application provides an antibody capable of specifically binding to CD3 or an antigen-binding fragment thereof, wherein the antibody or the antigen-binding fragment thereof comprises a heavy chain variable region (VH) comprising at least one, two or three complementarity-determining regions (CDRs) selected from the group consisting of:
(i) CDR-H1 having a sequence as shown in SEQ ID NO: 7, 13, 18, 24, 29, 35, 85, 86, 87 or 88, or a sequence that has one or more amino acid substitutions, deletions or additions (such as 1, 2 or 3 substitutions, deletions or additions) relative to any of the sequences;
(ii) CDR-H2 having a sequence as shown in SEQ ID NO: 8, 14, 19, 25, 30, 36, 51, 55 or 57, or a sequence that has one or more amino acid substitutions, deletions or additions (such as 1, 2 or 3 substitutions, deletions or additions) relative to any of the sequences; and
(iii) CDR-H3 having a sequence as shown in SEQ ID NO: 9, 15, 20, 26, 31, 37, 52, 56, 96, 97, 98, 99, 100, 101, 102, 103, 104 or 105, or a sequence that has one or more amino acid substitutions, deletions or additions (such as 1, 2 or 3 substitutions, deletions or additions) relative to any of the sequences;
   and/or the antibody or the antigen-binding fragment thereof comprises a light chain variable region (VL) comprising at least one, two or three CDRs selected from the group consisting of:
(iv) CDR-L1 having a sequence as shown in SEQ ID NO: 10, 16, 21, 27, 32, 38, 50, 53, 93 or 94, or a sequence that has one or more amino acid substitutions, deletions or additions (such as 1, 2 or 3 substitutions, deletions or additions) relative to any of the sequences;
(v) CDR-L2 having a sequence as shown in SEQ ID NO: 11, 17, 22, 28, 33, 39, 54, 58, 89, 90, 91, 92 or 95, or a sequence that has one or more amino acid substitutions, deletions or additions (such as 1, 2 or 3 substitutions, deletions or additions) relative to any of the sequences; and
(vi) CDR-L3 having a sequence as shown in SEQ ID NO: 12, 23 or 34, or a sequence that has one or more amino acid substitutions, deletions or additions (such as 1, 2 or 3 substitutions, deletions or additions) relative to any of the sequences.

In some preferred embodiments, the substitutions in any one of (i) to (vi) are conservative substitutions.

In some preferred embodiments, CDR-H1, CDR-H2 and CDR-H3 contained in the heavy chain variable region and/or CDR-L1, CDR-L2 and CDR-L3 contained in the light chain variable region are defined by a Kabat or IMGT numbering system. Table 4 in Example 5 illustrates amino acid sequences of CDRs of a murine antibody defined by the Kabat or IMGT numbering system.

In some preferred embodiments, the antibody or the antigen-binding fragment thereof comprises three VH CDRs and three VL CDRs selected from the following 33 groups:
(1) CDR-H1, CDR-H2, CDR-H3, CDR-L1, CDR-L2 and CDR-L3 having sequences as shown in SEQ ID NOs: 7, 8, 9, 10, 11 and 12 respectively or sequences that have one or more amino acid substitutions, deletions or additions (such as 1, 2 or 3 substitutions, deletions or additions) relative to any of the sequences;
(2) CDR-H1, CDR-H2, CDR-H3, CDR-L1, CDR-L2 and CDR-L3 having sequences as shown in SEQ ID NOs: 7, 51, 52, 53, 54 and 12 respectively or sequences that have one or more amino acid substitutions, deletions or additions (such as 1, 2 or 3 substitutions, deletions or additions) relative to any of the sequences;
(3) CDR-H1, CDR-H2, CDR-H3, CDR-L1, CDR-L2 and CDR-L3 having sequences as shown in SEQ ID NOs: 7, 51, 52, 10, 11 and 12 respectively or sequences that have one or more amino acid substitutions, deletions or additions (such as 1, 2 or 3 substitutions, deletions or additions) relative to any of the sequences;
(4) CDR-H1, CDR-H2, CDR-H3, CDR-L1, CDR-L2 and CDR-L3 having sequences as shown in SEQ ID NOs: 13, 14, 15, 16, 17 and 12 respectively or sequences that have one or more amino acid substitutions, deletions or additions (such as 1, 2 or 3 substitutions, deletions or additions) relative to any of the sequences;
(5) CDR-H1, CDR-H2, CDR-H3, CDR-L1, CDR-L2 and CDR-L3 having sequences as shown in SEQ ID NOs: 13, 55, 56, 16, 17 and 12 respectively or sequences that have one or more amino acid substitutions, deletions or additions (such as 1, 2 or 3 substitutions, deletions or additions) relative to any of the sequences;
(6) CDR-H1, CDR-H2, CDR-H3, CDR-L1, CDR-L2 and CDR-L3 having sequences as shown in SEQ ID NOs: 18, 19, 20, 21, 22 and 23 respectively or sequences that have one or more amino acid substitutions, deletions or additions (such as 1, 2 or 3 substitutions, deletions or additions) relative to any of the sequences;
(7) CDR-H1, CDR-H2, CDR-H3, CDR-L1, CDR-L2 and CDR-L3 having sequences as shown in SEQ ID NOs: 18, 57, 20, 21, 58 and 23 respectively or sequences that have one or more amino acid substitutions, deletions or additions (such as 1, 2 or 3 substitutions, deletions or additions) relative to any of the sequences;
(8) CDR-H1, CDR-H2, CDR-H3, CDR-L1, CDR-L2 and CDR-L3 having sequences as shown in SEQ ID NOs: 24, 25, 26, 27, 28 and 23 respectively or sequences that have one or more amino acid substitutions, deletions or additions (such as 1, 2 or 3 substitutions, deletions or additions) relative to any of the sequences;
(9) CDR-H1, CDR-H2, CDR-H3, CDR-L1, CDR-L2 and CDR-L3 having sequences as shown in SEQ ID NOs: 29, 30, 31, 32, 33 and 34 respectively or sequences that have one or more amino acid substitutions, deletions or additions (such as 1, 2 or 3 substitutions, deletions or additions) relative to any of the sequences;
(10) CDR-H1, CDR-H2, CDR-H3, CDR-L1, CDR-L2 and CDR-L3 having sequences as shown in SEQ ID NOs: 29, 30, 31, 50, 33 and 34 respectively or sequences that have one or more amino acid substitutions, deletions or additions (such as 1, 2 or 3 substitutions, deletions or additions) relative to any of the sequences;
(11) CDR-H1, CDR-H2, CDR-H3, CDR-L1, CDR-L2 and CDR-L3 having sequences as shown in SEQ ID NOs: 35, 36, 37, 38, 39 and 34 respectively or sequences that have one or more amino acid substitutions, deletions or additions (such as 1, 2 or 3 substitutions, deletions or additions) relative to any of the sequences;
(12) CDR-H1, CDR-H2, CDR-H3, CDR-L1, CDR-L2 and CDR-L3 having sequences as shown in SEQ ID NOs: 87, 57, 20, 21, 58 and 23 respectively or sequences that have one or more amino acid substitutions, deletions or additions (such as 1, 2 or 3 substitutions, deletions or additions) relative to any of the sequences;
(13) CDR-H1, CDR-H2, CDR-H3, CDR-L1, CDR-L2 and CDR-L3 having sequences as shown in SEQ ID NOs: 18, 57, 20, 21, 89 and 23 respectively or sequences that have one or more amino acid substitutions, deletions or additions (such as 1, 2 or 3 substitutions, deletions or additions) relative to any of the sequences;
(14) CDR-H1, CDR-H2, CDR-H3, CDR-L1, CDR-L2 and CDR-L3 having sequences as shown in SEQ ID NOs: 18, 57, 20, 21, 91 and 23 respectively or sequences that have one or more amino acid substitutions, deletions or additions (such as 1, 2 or 3 substitutions, deletions or additions) relative to any of the sequences;
(15) CDR-H1, CDR-H2, CDR-H3, CDR-L1, CDR-L2 and CDR-L3 having sequences as shown in SEQ ID NOs: 18, 57, 20, 93, 58 and 23 respectively or sequences that have one or more amino acid substitutions, deletions or additions (such as 1, 2 or 3 substitutions, deletions or additions) relative to any of the sequences;
(16) CDR-H1, CDR-H2, CDR-H3, CDR-L1, CDR-L2 and CDR-L3 having sequences as shown in SEQ ID NOs: 18, 57, 20, 21, 95 and 23 respectively or sequences that have one or more amino acid substitutions, deletions or additions (such as 1, 2 or 3 substitutions, deletions or additions) relative to any of the sequences;
(17) CDR-H1, CDR-H2, CDR-H3, CDR-L1, CDR-L2 and CDR-L3 having sequences as shown in SEQ ID NOs: 18, 57, 96, 21, 58 and 23 respectively or sequences that have one or more amino acid substitutions, deletions or additions (such as 1, 2 or 3 substitutions, deletions or additions) relative to any of the sequences;
(18) CDR-H1, CDR-H2, CDR-H3, CDR-L1, CDR-L2 and CDR-L3 having sequences as shown in SEQ ID NOs: 18, 57, 98, 21, 58 and 23 respectively or sequences that have one or more amino acid substitutions, deletions or additions (such as 1, 2 or 3 substitutions, deletions or additions) relative to any of the sequences;
(19) CDR-H1, CDR-H2, CDR-H3, CDR-L1, CDR-L2 and CDR-L3 having sequences as shown in SEQ ID NOs: 18, 57, 100, 21, 58 and 23 respectively or sequences that have one or more amino acid substitutions, deletions or additions (such as 1, 2 or 3 substitutions, deletions or additions) relative to any of the sequences;
(20) CDR-H1, CDR-H2, CDR-H3, CDR-L1, CDR-L2 and CDR-L3 having sequences as shown in SEQ ID NOs: 18, 57, 102, 21, 58 and 23 respectively or sequences that have one or more amino acid substitutions, deletions or additions (such as 1, 2 or 3 substitutions, deletions or additions) relative to any of the sequences;
(21) CDR-H1, CDR-H2, CDR-H3, CDR-L1, CDR-L2 and CDR-L3 having sequences as shown in SEQ ID NOs: 18, 57, 104, 21, 58 and 23 respectively or sequences that have one or more amino acid substitutions, deletions or additions (such as 1, 2 or 3 substitutions, deletions or additions) relative to any of the sequences;
(22) CDR-H1, CDR-H2, CDR-H3, CDR-L1, CDR-L2 and CDR-L3 having sequences as shown in SEQ ID NOs: 85, 25, 26, 27, 28 and 23 respectively or sequences that have one or more amino acid substitutions, deletions or additions (such as 1, 2 or 3 substitutions, deletions or additions) relative to any of the sequences;
(23) CDR-H1, CDR-H2, CDR-H3, CDR-L1, CDR-L2 and CDR-L3 having sequences as shown in SEQ ID NOs: 86, 25, 26, 27, 28 and 23 respectively or sequences that have one or more amino acid substitutions, deletions or additions (such as 1, 2 or 3 substitutions, deletions or additions) relative to any of the sequences;
(24) CDR-H1, CDR-H2, CDR-H3, CDR-L1, CDR-L2 and CDR-L3 having sequences as shown in SEQ ID NOs: 88, 25, 26, 27, 28 and 23 respectively or sequences that have one or more amino acid substitutions, deletions or additions (such as 1, 2 or 3 substitutions, deletions or additions) relative to any of the sequences;
(25) CDR-H1, CDR-H2, CDR-H3, CDR-L1, CDR-L2 and CDR-L3 having sequences as shown in SEQ ID NOs: 24, 25, 26, 27, 90 and 23 respectively or sequences that have one or more amino acid substitutions, deletions or additions (such as 1, 2 or 3 substitutions, deletions or additions) relative to any of the sequences;
(26) CDR-H1, CDR-H2, CDR-H3, CDR-L1, CDR-L2 and CDR-L3 having sequences as shown in SEQ ID NOs: 24, 25, 26, 27, 92 and 23 respectively or sequences that have one or more amino acid substitutions, deletions or additions (such as 1, 2 or 3 substitutions, deletions or additions) relative to any of the sequences;
(27) CDR-H1, CDR-H2, CDR-H3, CDR-L1, CDR-L2 and CDR-L3 having sequences as shown in SEQ ID NOs: 86, 25, 26, 27, 92 and 23 respectively or sequences that have one or more amino acid substitutions, deletions or additions (such as 1, 2 or 3 substitutions, deletions or additions) relative to any of the sequences;
(28) CDR-H1, CDR-H2, CDR-H3, CDR-L1, CDR-L2 and CDR-L3 having sequences as shown in SEQ ID NOs: 24, 25, 26, 94, 28 and 23 respectively or sequences that have one or more amino acid substitutions, deletions or additions (such as 1, 2 or 3 substitutions, deletions or additions) relative to any of the sequences;
(29) CDR-H1, CDR-H2, CDR-H3, CDR-L1, CDR-L2 and CDR-L3 having sequences as shown in SEQ ID NOs: 24, 25, 97, 27, 28 and 23 respectively or sequences that have one or more amino acid substitutions, deletions or additions (such as 1, 2 or 3 substitutions, deletions or additions) relative to any of the sequences;
(30) CDR-H1, CDR-H2, CDR-H3, CDR-L1, CDR-L2 and CDR-L3 having sequences as shown in SEQ ID NOs: 24, 25, 99, 27, 28 and 23 respectively or sequences that have one or more amino acid substitutions, deletions or additions (such as 1, 2 or 3 substitutions, deletions or additions) relative to any of the sequences;
(31) CDR-H1, CDR-H2, CDR-H3, CDR-L1, CDR-L2 and CDR-L3 having sequences as shown in SEQ ID NOs: 24, 25, 101, 27, 28 and 23 respectively or sequences that have one or more amino acid substitutions, deletions or additions (such as 1, 2 or 3 substitutions, deletions or additions) relative to any of the sequences;
(32) CDR-H1, CDR-H2, CDR-H3, CDR-L1, CDR-L2 and CDR-L3 having sequences as shown in SEQ ID NOs: 24, 25, 103, 27, 28 and 23 respectively or sequences that have one or more amino acid substitutions, deletions or additions (such as 1, 2 or 3 substitutions, deletions or additions) relative to any of the sequences; and
(33) CDR-H1, CDR-H2, CDR-H3, CDR-L1, CDR-L2 and CDR-L3 having sequences as shown in SEQ ID NOs: 24, 25, 105, 27, 28 and 23 respectively or sequences that have one or more amino acid substitutions, deletions or additions (such as 1, 2 or 3 substitutions, deletions or additions) relative to any of the sequences.

In some embodiments, the antibody or the antigen-binding fragment thereof is murine or chimeric, and the heavy chain variable region thereof comprises a heavy chain FR region of murine IgG1, IgG2 or IgG3 or a variant thereof; and the light chain variable region thereof comprises a light chain FR region of a murine κ or λ chain or a variant thereof. The numbers of preferred amino acid sequences of variable regions of the murine antibody are given in Example 5.

In some preferred embodiments, the murine or chimeric antibody or the antigen-binding fragment thereof comprises VH and VL sequences selected from the following three groups:
(i) a VH domain comprising an amino acid sequence as shown in SEQ ID NO: 1 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 1; and a VL domain comprising an amino acid sequence as shown in SEQ ID NO: 2 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 2;
(ii) a VH domain comprising an amino acid sequence as shown in SEQ ID NO: 3 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 3; and a VL domain comprising an amino acid sequence as shown in SEQ ID NO: 4 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 4; and
(iii) a VH domain comprising an amino acid sequence as shown in SEQ ID NO: 5 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 5; and a VL domain comprising an amino acid sequence as shown in SEQ ID NO: 6 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 6.

In some embodiments, the antibody or the antigen-binding fragment thereof is humanized. A basic flow of a humanization strategy is given in Example 6, and the numbers of preferred amino acid sequences of variable regions of a humanized antibody are given in Table 3.

In some preferred embodiments, the humanized antibody or the antigen-binding fragment thereof comprises VH and VL sequences selected from the group consisting of:
(1) a VH domain comprising an amino acid sequence as shown in SEQ ID NO: 40 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 40; and a VL domain comprising an amino acid sequence as shown in SEQ ID NO: 41 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 41;
(2) a VH domain comprising an amino acid sequence as shown in SEQ ID NO: 42 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 42; and a VL domain comprising an amino acid sequence as shown in SEQ ID NO: 43 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 43;
(3) a VH domain comprising an amino acid sequence as shown in SEQ ID NO: 44 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 44; and a VL domain comprising an amino acid sequence as shown in SEQ ID NO: 45 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 45;
(4) a VH domain comprising an amino acid sequence as shown in SEQ ID NO: 46 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 46; and a VL domain comprising an amino acid sequence as shown in SEQ ID NO: 47 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 47;
(5) a VH domain comprising an amino acid sequence as shown in SEQ ID NO: 48 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 48; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(6) a VH domain comprising an amino acid sequence as shown in SEQ ID NO: 106 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 106; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(7) a VH domain comprising an amino acid sequence as shown in SEQ ID NO: 107 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 107; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(8) a VH domain comprising an amino acid sequence as shown in SEQ ID NO: 108 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 108; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(9) a VH domain comprising an amino acid sequence as shown in SEQ ID NO: 48 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 48; and a VL domain comprising an amino acid sequence as shown in SEQ ID NO: 109 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 109;
(10) a VH domain comprising an amino acid sequence as shown in SEQ ID NO: 48 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 48; and a VL domain comprising an amino acid sequence as shown in SEQ ID NO: 110 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 110;
(11) a VH domain comprising an amino acid sequence as shown in SEQ ID NO: 111 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 111; and a VL domain comprising an amino acid sequence as shown in SEQ ID NO: 112 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 112;
(12) a VH domain comprising an amino acid sequence as shown in SEQ ID NO: 48 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 48; and a VL domain comprising an amino acid sequence as shown in SEQ ID NO: 113 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 113;
(13) a VH domain comprising an amino acid sequence as shown in SEQ ID NO: 48 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 48; and a VL domain comprising an amino acid sequence as shown in SEQ ID NO: 114 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 114;
(14) a VH domain comprising an amino acid sequence as shown in SEQ ID NO: 115 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 115; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(15) a VH domain comprising an amino acid sequence as shown in SEQ ID NO: 116 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 116; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(16) a VH domain comprising an amino acid sequence as shown in SEQ ID NO: 117 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 117; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(17) a VH domain comprising an amino acid sequence as shown in SEQ ID NO: 118 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 118; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49; and
(18) a VH domain comprising an amino acid sequence as shown in SEQ ID NO: 119 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 119; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49.

In some embodiments, the antibody or the antigen-binding fragment thereof in the present application further comprises a constant region sequence derived from a mammalian (for example, murine or human) immunoglobulin or a variant thereof, wherein the variant has one or more substitutions, deletions or additions relative to a sequence from which the variant is derived. In some preferred embodiments, the variant has one or more conservative substitutions relative to the sequence from which the variant is derived. In some embodiments, an anti-CD3 antibody molecule has a heavy chain constant region (Fc) selected from, for example, heavy chain constant regions of IgG1, IgG2, IgG3, IgG4, IgM, IgA1, IgA2, IgD and IgE, particularly selected from, for example, heavy chain constant regions of IgG1, IgG2, IgG3 and IgG4, and more particularly selected from a heavy chain constant region of IgG1, IgG2 or IgG4 (such as human IgG1, IgG2 or IgG4). In some embodiments, the anti-CD3 antibody molecule has a light chain constant region selected from, for example, κ or λ light chain constant regions, preferably a κ light chain constant region (such as a human κ light chain).

In some embodiments, the constant region is changed, for example, mutated to modify properties of the anti-CD3 antibody molecule (for example, to change one or more of the following properties: Fc receptor binding, antibody glycosylation, the number of cysteine residues, a function of an effector cell or a function of a complement). At least one amino acid residue in the constant region of the antibody may be replaced with different residues for a functional change, for example, changing the affinity of the antibody to an effector ligand (such as FcR or complement C1q), thereby changing (such as enhancing, decreasing or eliminating) an effector function. A method for replacing amino acid residues in the Fc region of the antibody to change the effector function is known in the art (referring to, for example, EP388151A1, US5648260 and US5624821). The Fc region of the antibody mediates several important effector functions, such as ADCC, phagocytosis and CDC. In some cases, these effector functions are necessary for therapeutic antibodies; but in other cases, these effector functions may be unnecessary or even harmful, which depends on an intended purpose. Therefore, in some embodiments, the antibody or the antigen-binding fragment thereof in the present application has decreased or even eliminated effector functions (such as ADCC and/or CDC activity). An amino acid mutation in human IgG4 that stabilizes the structure of the antibody, such as S228P (in EU nomenclature, or S241P in Kabat nomenclature), is also contemplated.

In such embodiments, the antibody or the antigen-binding fragment thereof in the present application comprises a variant of a heavy chain constant region of human IgG, wherein the variant has at least one of the following substitutions relative to a wild-type sequence from which the variant is derived: Ser228Pro, Leu234Ala, Leu235Ala, Gly237Ala, Asp265Ala, Asn297Ala, Pro329Ala, Asp356Glu and Leu358Met (these amino acid positions are positions based on an EU numbering system, Edelman GM et al, Proc Natl Acad USA, 63, 78-85 (1969). PMID: 5257969).

In some example embodiments, the antibody or the antigen-binding fragment thereof in the present application comprises a variant of a heavy chain constant region of human IgG1, wherein the variant has the following substitution relative to a wild-type sequence from which the variant is derived: Leu234Ala, Leu235Ala (positions based on the EU numbering system). In such embodiments, the antibody or the antigen-binding fragment thereof in the present application has decreased ADCC activity and CDC activity.

In some example embodiments, the antibody or the antigen-binding fragment thereof in the present application comprises a variant of a heavy chain constant region of human IgG1, wherein the variant has the following substitution relative to a wild-type sequence from which the variant is derived: Asn297Ala (a position based on the EU numbering system). In such embodiments, the antibody or the antigen-binding fragment thereof in the present application has eliminated ADCC activity.

In some example embodiments, the antibody or the antigen-binding fragment thereof in the present application comprises a variant of a heavy chain constant region of human IgG1, wherein the variant has the following substitution relative to a wild-type sequence from which the variant is derived: Asp265Ala, Pro329Ala (positions based on the EU numbering system). In such embodiments, the antibody or the antigen-binding fragment thereof in the present application has eliminated ADCC activity.

In some example embodiments, the antibody or the antigen-binding fragment thereof in the present application comprises a variant of a heavy chain constant region of human IgG4, wherein the variant has the following substitution relative to a wild-type sequence from which the variant is derived: Ser228Pro (a position based on the EU numbering system). In such embodiments, the antibody or the antigen-binding fragment thereof in the present application is stable in structure, can reduce Fab-arm exchange, and is not easy to produce an incomplete antibody.

In some preferred embodiments, a heavy chain of the antibody has an amino acid sequence as shown in SEQ ID NO: 65, a sequence that has one or more substitutions, deletions or additions (such as 1, 2, 3, 4 or 5 substitutions, deletions or additions) relative to SEQ ID NO: 65 or a sequence that has at least 80%, at least 85%, at least 90%, at least 91%, at least 92%, at least 93%, at least 94%, at least 95%, at least 96%, at least 97%, at least 98%, at least 99% or higher identity to SEQ ID NO: 65; and/or a light chain of the antibody has an amino acid sequence as shown in SEQ ID NO: 66, a sequence that has one or more substitutions, deletions or additions (such as 1, 2, 3, 4 or 5 substitutions, deletions or additions) relative to SEQ ID NO: 66 or a sequence that has at least 80%, at least 85%, at least 90%, at least 91%, at least 92%, at least 93%, at least 94%, at least 95%, at least 96%, at least 97%, at least 98%, at least 99% or higher identity to SEQ ID NO: 66.

In some preferred embodiments, a heavy chain of the antibody has an amino acid sequence as shown in SEQ ID NO: 69, a sequence that has one or more substitutions, deletions or additions (such as 1, 2, 3, 4 or 5 substitutions, deletions or additions) relative to SEQ ID NO: 69 or a sequence that has at least 80%, at least 85%, at least 90%, at least 91%, at least 92%, at least 93%, at least 94%, at least 95%, at least 96%, at least 97%, at least 98%, at least 99% or higher identity to SEQ ID NO: 69; and/or a light chain of the antibody has an amino acid sequence as shown in SEQ ID NO: 70, a sequence that has one or more substitutions, deletions or additions (such as 1, 2, 3, 4 or 5 substitutions, deletions or additions) relative to SEQ ID NO: 70 or a sequence that has at least 80%, at least 85%, at least 90%, at least 91%, at least 92%, at least 93%, at least 94%, at least 95%, at least 96%, at least 97%, at least 98%, at least 99% or higher identity to SEQ ID NO: 70.

In some preferred embodiments, a heavy chain of the antibody has an amino acid sequence as shown in SEQ ID NO: 73, a sequence that has one or more substitutions, deletions or additions (such as 1, 2, 3, 4 or 5 substitutions, deletions or additions) relative to SEQ ID NO: 73 or a sequence that has at least 80%, at least 85%, at least 90%, at least 91%, at least 92%, at least 93%, at least 94%, at least 95%, at least 96%, at least 97%, at least 98%, at least 99% or higher identity to SEQ ID NO: 73; and/or a light chain of the antibody has an amino acid sequence as shown in SEQ ID NO: 74, a sequence that has one or more substitutions, deletions or additions (such as 1, 2, 3, 4 or 5 substitutions, deletions or additions) relative to SEQ ID NO: 74 or a sequence that has at least 80%, at least 85%, at least 90%, at least 91%, at least 92%, at least 93%, at least 94%, at least 95%, at least 96%, at least 97%, at least 98%, at least 99% or higher identity to SEQ ID NO: 74.

In some preferred embodiments, a heavy chain of the antibody has an amino acid sequence as shown in SEQ ID NO: 77, a sequence that has one or more substitutions, deletions or additions (such as 1, 2, 3, 4 or 5 substitutions, deletions or additions) relative to SEQ ID NO: 77 or a sequence that has at least 80%, at least 85%, at least 90%, at least 91%, at least 92%, at least 93%, at least 94%, at least 95%, at least 96%, at least 97%, at least 98%, at least 99% or higher identity to SEQ ID NO: 77; and/or a light chain of the antibody has an amino acid sequence as shown in SEQ ID NO: 78, a sequence that has one or more substitutions, deletions or additions (such as 1, 2, 3, 4 or 5 substitutions, deletions or additions) relative to SEQ ID NO: 78 or a sequence that has at least 80%, at least 85%, at least 90%, at least 91%, at least 92%, at least 93%, at least 94%, at least 95%, at least 96%, at least 97%, at least 98%, at least 99% or higher identity to SEQ ID NO: 78.

In some preferred embodiments, a heavy chain of the antibody has an amino acid sequence as shown in SEQ ID NO: 81, a sequence that has one or more substitutions, deletions or additions (such as 1, 2, 3, 4 or 5 substitutions, deletions or additions) relative to SEQ ID NO: 81 or a sequence that has at least 80%, at least 85%, at least 90%, at least 91%, at least 92%, at least 93%, at least 94%, at least 95%, at least 96%, at least 97%, at least 98%, at least 99% or higher identity to SEQ ID NO: 81; and/or a light chain of the antibody has an amino acid sequence as shown in SEQ ID NO: 82, a sequence that has one or more substitutions, deletions or additions (such as 1, 2, 3, 4 or 5 substitutions, deletions or additions) relative to SEQ ID NO: 82 or a sequence that has at least 80%, at least 85%, at least 90%, at least 91%, at least 92%, at least 93%, at least 94%, at least 95%, at least 96%, at least 97%, at least 98%, at least 99% or higher identity to SEQ ID NO: 82.

In some relatively preferred embodiments, the above substitutions are conservative substitutions.

In some preferred embodiments, the antibody or the antigen-binding fragment thereof in the present application is a chimeric antibody or a humanized antibody. In some preferred embodiments, the antibody or the antigen-binding fragment thereof in the present application is selected from scFv, Fab, Fab', (Fab')2, an Fv fragment, a diabody, a bispecific antibody or a multi-specific antibody.

The antibody or the antigen-binding fragment thereof in the present application has high specificity and high affinity to CD3 (particularly human CD3). In some preferred embodiments, the antibody or the antigen-binding fragment thereof in the present application can bind to CD3 (particularly human CD3) at a KD of about 1 nM or less.

In a second aspect, the present application discloses a nucleotide sequence encoding the anti-CD3 antibody molecule of the present application. In some embodiments, the nucleotide sequence encoding the anti-CD3 antibody molecule is codon-optimized. For example, the present application is characterized by a first nucleic acid and a second nucleic acid encoding the heavy chain variable region and the light chain variable region of the anti-CD3 antibody molecule, respectively, wherein the antibody molecule is selected from any one of: AP191, AP591, AP831, AB610, AB611, AB612, AB613, AB614 or a sequence substantially identical thereto. For example, the nucleic acid may comprise nucleotide sequences of AB610, AB611, AB612, AB613 and AB614 shown in Table 6 or sequences substantially identical thereto (for example, sequences that have at least about 85%, 90%, 95%, 99% or more similarity, sequences that have one or more nucleotide substitutions (such as conservative substitutions) or sequences that differ from the sequences shown in Table 6 by no more than 3, 6, 15, 30 or 45 nucleotides).

In preferred embodiments of the present application, a DNA molecule encoding a heavy chain of the antibody has a nucleotide sequence as shown in SEQ ID NO: 67, 71, 75, 79 or 83, and a DNA molecule encoding a light chain of the antibody has a nucleotide sequence as shown in SEQ ID NO: 68, 72, 76, 80 or 84.

In a third aspect of the present application, the present application provides a vector (such as a cloning vector or an expression vector), which comprises the isolated nucleic acid molecule of the present application. In some preferred embodiments, the vector of the present application is, for example, a plasmid, a cosmid or a phage. In some preferred embodiments, the vector can express the antibody or the antigen-binding fragment thereof in the present application in a subject (for example, a mammal such as a human).

In a fourth aspect of the present application, the present application provides a host cell, which comprises the isolated nucleic acid molecule of the present application or the vector of the present application. The host cell may be a eukaryotic cell (for example, a mammalian cell, an insect cell, a yeast cell) or a prokaryotic cell (for example, *Escherichia coli*)*.* Suitable eukaryotic cells include, but are not limited to, NS0 cells, Vero cells, Hela cells, COS cells, CHO cells, HEK293 cells, BHK cells and MDCKII cells. Suitable insect cells include, but are not limited to, Sf9 cells. In some preferred embodiments, the host cell of the present application is a mammalian cell, such as CHO (for example, CHO-K1, CHO-S, CHO DXB11 and CHO DG44). In a fifth aspect, the present application discloses a pharmaceutical composition, which comprises the anti-CD3 antibody molecule of the present application and a pharmaceutically acceptable carrier and/or excipient and/or stabilizer.

In some preferred embodiments, the pharmaceutical composition may further include an additional pharmaceutically active agent. In some preferred embodiments, the additional pharmaceutically active agent is a medicament with anti-tumor activity. In some preferred embodiments, the additional pharmaceutically active agent is a medicament for treating an organ transplantation rejection. In some preferred embodiments, the additional pharmaceutically active agent is a medicament for treating an autoimmune disease.

In some preferred embodiments, in the pharmaceutical composition, the antibody or the antigen-binding fragment thereof in the present application and the additional pharmaceutically active agent are provided as separate components or as components of the same composition. Therefore, the antibody or the antigen-binding fragment thereof in the present application and the additional pharmaceutically active agent may be administered simultaneously, separately or sequentially.

In a sixth aspect, the present application further provides a method for preparing the antibody or the antigen-binding fragment thereof in the present application. The method comprises: (a) obtaining genes of the antibody or the antigen-binding fragment thereof, and constructing an expression vector of the antibody or the antigen-binding fragment thereof; (b) transfecting the expression vector into a host cell by a genetic engineering method; (c) culturing the host cell under a condition that allows the antibody or the antigen-binding fragment thereof to be produced; (d) separating and purifying the produced antibody or antigen-binding fragment thereof.

The expression vector in step (a) is one or more selected from a plasmid, a bacterium and a virus; preferably, the expression vector is pcDNA3.1.

The host cell into which the constructed vector is transfected by the genetic engineering method in step (b) includes a prokaryotic cell, a yeast cell or a mammalian cell, such as a CHO cell, an NS0 cell or another mammalian cell, preferably a CHO cell.

The antibody or the antigen-binding fragment thereof is separated and purified in step (d) by a conventional immunoglobulin purification method comprising protein A affinity chromatography and ion exchange, hydrophobic chromatography or molecular sieve.

In a seventh aspect of the present application, the present application relates to use of the antibody or the antigen-binding fragment thereof in the present application for preparing a medicament for:
(1) activating or inhibiting the activity of T cells *in vitro* or *in vivo* of a subject (such as a human);
(2) treating a tumor in a subject (such as a human);
(3) treating an organ transplantation rejection in a subject (such as a human); or
(4) treating an autoimmune disease in a subject (such as a human).

In some embodiments, the tumor is selected from a solid tumor, a hematological tumor (such as leukemia, lymphoma and myeloma such as multiple myeloma) and a metastatic disease. More special examples of cancer include, but are not limited to, lung cancer (for example, lung adenocarcinoma or non-small cell lung cancer (NSCLC) (for example, NSCLC with a history of squamous and/or non-squamous disease, or NSCLC adenocarcinoma)), melanoma (for example, advanced melanoma), renal cancer (for example, renal cell carcinoma), liver cancer (for example, hepatocellular carcinoma), myeloma (for example, multiple myeloma), prostate cancer, breast cancer (for example, breast cancer that does not express one, two or all of an estrogen receptor, a progesterone receptor or Her2/neu, for example, triple negative breast cancer), ovarian cancer, colorectal cancer, pancreatic cancer, head and neck cancer (for example, head and neck squamous cell carcinoma (HNSCC)), anal cancer, gastro-esophageal cancer (for example, esophageal squamous cell carcinoma), mesothelioma, nasopharyngeal cancer, thyroid cancer, cervical cancer, a lymphoproliferative disease (for example, post-transplant lymphoproliferative disease) or hematological cancer (for example diffuse large B-cell lymphoma, T-cell lymphoma, B-cell lymphoma or non-Hodgkin's lymphoma) or leukemia (for example, myeloid leukemia or lymphocytic leukemia).

In some embodiments, the organ transplantation rejection is selected from abnormal or undesired immune responses associated with a cell, a tissue or organ transplantation such as kidney, liver and heart transplantation, for example, a graft-versus-host disease (GVHD) or reducing a risk of an allograft rejection.

In some embodiments, the autoimmune disease is selected from rheumatoid arthritis, juvenile rheumatoid arthritis, psoriasis, systemic lupus erythematosus, primary biliary cirrhosis, autoimmune hemolytic anemia, aplastic anemia, autoimmune thrombocytopenic purpura, idiopathic thrombocytopenic purpura, diabetes, encephalomyelitis, Graves' disease, myasthenia gravis, Wegener's disease, autoimmune hepatitis and multiple sclerosis.

In an eighth aspect, the present application provides any bispecific molecule comprising the antibody of the present application. For example, the preceding CD3 antibody may be functionally linked to an antibody or an antibody fragment having another antigen-binding characteristic to form a bispecific antibody. For example, the bispecific antibody includes, but is not limited to, an antibody against a molecule selected from the group consisting of CD19, CD20, CD22, CD25, CD30, CD33, CD38, CD39, CD40, CD47, CD52, CD73, CD74, CD123, CD133, CD138, BCMA, CA125, CEA, CS1, DLL3, DLL4, EGFR, EpCAM, FLT3, gpA33, GPC-3, Her2, MEGE-A3, NYESO1, PSMA, TAG-72, CIX, folate-binding protein, GD2, GD3, GM2, VEGF, VEGFR2, VEGFR3, cadherin, integrin, mesothelin, Claudin18, αVβ3, α5β1, ERBB3, c-MET, IGF1R, EPHA3, TRAILR1, TRAILR2, RANKL, a B7 protein family, a mucin family, FAP and tenascin.

In a ninth aspect, the present application provides a method for treating (for example, inhibiting and/or delaying development) a tumor, an organ transplantation rejection or an autoimmune disease in a subject. The method comprises: administering the subject with the anti-CD3 antibody molecule described herein, for example, a therapeutically effective amount of the anti-CD3 antibody molecule, alone or in combination with one or more active agents or procedures.

In a tenth aspect, the present application provides a diagnostic or therapeutic kit, which comprises the anti-CD3 antibody molecule described in the present application and instructions for use.

The humanized anti-CD3 antibody prepared in the present application binds to CD3 with high affinity and extremely strong specificity. *In vitro* biological study data show that the humanized antibody provided in the present application can significantly promote T cell proliferation and activate T cells, thereby attenuating and avoiding excessive cytokine storms. Additionally, the antibody of the present application is expressed in CHO cells and has the advantages of a high yield, high activity, a simple purification process and a low production cost.

### Abbreviations and definitions

The following abbreviations are used herein:
- CDR: Complementarity-determining region in a variable region of an immunoglobulin
- FR: Framework region of an antibody: amino acid residues excluding CDR residues in a variable region of the antibody
- IgG: Immunoglobulin G
- IMGT: Numbering system based on the international ImMunoGeneTics information system ^{®} (IMGT) initiated by Lefranc et al., referring to Lefranc et al., Dev. Comparat. Immunol. 27: 55-77, 2003.
- mAb: Monoclonal antibody
- EC₅₀: A concentration at which 50% efficacy or binding is generated
- IC₅₀: A concentration at which 50% inhibition is generated
- ELISA: Enzyme-linked immunosorbent assay
- PCR: Polymerase chain reaction
- HRP: Horseradish peroxidase
- IL-2: Interleukin 2
- K_{D}: Equilibrium dissociation constant
- ka: Association rate constant
- kd: Dissociation rate constant

In the present application, unless otherwise specified, the scientific and technical terms used herein have meanings generally understood by those skilled in the art. Moreover, operation steps used herein, such as cell culture, biochemistry, nucleic acid chemistry and immunology laboratory, are all conventional steps widely used in the art. Meanwhile, for a better understanding of the present application, the definitions and explanations of related terms are provided below.

Eu in the term "EU Numbering System or Scheme" refers to the first human immunoglobulin IgG1 separated and purified by Gerald M Edelman et al. at the end of the 1960s (1968-1969), where the amino acid sequence of IgG1 was determined and numbered (Edelman GM et al, 1969, Proc Natl Acad USA, 63: 78-85). A heavy chain constant region of another immunoglobulin is aligned with the amino acid sequence of Eu, and the corresponding amino acid positions are Eu numbers. The Eu numbering system is mainly directed to a heavy chain constant region of an immunoglobulin, including CH1, CH2, CH3 and a hinge region.

The term "Kabat Numbering System or Scheme" refers to a standardized numbering scheme of variable regions of human immunoglobulins firstly proposed by Kabat *et al.* in 1979 (Kabat EA, Wu TT, Bilofsky H, Sequences of Immunoglobulin Chains: Tabulation and Analysis of Amino Acid Sequences of Precursors, V-regions, C-regions, J-Chain and β2-Microglobulins. 1979. Department of Health, Education, and Welfare, Public Health Service, National Institutes of Health). In the book "Sequences of Proteins of Immunological Interest" (Kabat EA, Wu TT, Perry HM, Gottesman KS, Foeller C. 1991. Sequences of Proteins of Immunological Interest, 5th edition. Bethesda, MD: US Department of Health and Human Services, National Institutes for Health), Kabat *et al.* compared and numbered the amino acid sequences of light and heavy chains of an antibody. They have found that these analyzed sequences exhibit variable lengths and default and inserted amino acids or amino acid fragments can only be present at particular positions. Interestingly, insertion points are mostly located in CDRs but may also appear at some positions of a framework region. In the Kabat numbering scheme, a light chain variable region is numbered to position 109, a heavy chain variable region is numbered to position 113, and inserted amino acids of the light and heavy chains are identified and annotated by letters (for example, 27a, 27b, ...). No Lambda light chain comprises a residue at position 10, whereas Lambda and Kappa light chains are encoded by two different genes and located on different chromosomes. The Lambda and Kappa light chains may be distinguished by differences in the amino acid sequences of their constant regions. Different from the EU numbering system directed to the heavy chain constant region only, the Kabat numbering system has a numbering range covering a full-length immunoglobulin sequence, including variable and constant regions of the light and heavy chains of the immunoglobulin.

The term "binding" defines the affinity interaction between a specific epitope on an antigen and its corresponding antibody and is generally understood as "specific recognition". "Specific recognition" means that the antibody of the present application does not or substantially does not have cross-reaction with any polypeptide other than the target antigen. The degree of specificity may be determined by immunological techniques, including, but not limited to, immunoblotting, immunoaffinity chromatography and flow cytometry. In the present application, the specific recognition is preferably determined by flow cytometry, and the criteria for the specific recognition in specific cases can be determines by those of ordinary skill in the art according to the general knowledge of the art which they know.

The term "antigen" is a foreign substance capable of eliciting antibody production by an organism itself or by a human and is any substance capable of eliciting an immune response, such as bacteria and viruses. Foreign antigen molecules are recognized and processed by B cells or antigen-presenting cells (for example, macrophages, dendritic cells, endothelial cells and B cells) and bind to a major histocompatibility complex (for example, MHC II molecules) to form a complex to activate T cells, initiating a continuous immune response.

The term "antibody" generally refers to proteinaceous binding molecules with immunoglobulin-like functions. Typical examples of the antibody are immunoglobulins and their derivatives or functional fragments as long as they exhibit the desired binding specificity. Techniques for preparing the antibody are well known in the art. The "antibody" includes different classes of natural immunoglobulins (such as IgA, IgG, IgM, IgD and IgE) and subclasses (such as IgG1, IgG2, IgA1 and IgA2). The "antibody" further includes non-natural immunoglobulins which include, for example, a single-chain antibody, a chimeric antibody (for example, humanized murine antibody) and a heterologous conjugated antibody (for example, bispecific antibody) as well as antigen-binding fragments thereof (for example, Fab', F(ab')₂, Fab, Fv and rIgG). See, for example, Pierce Catalog and Handbook, 1994-1995, Pierce Chemical Co., Rockford, Ill; Kuby, J., Immunology, 3rd Ed., WH Freeman & Co, New York, 1997. The antibody can bind to one antigen to be "monospecific", bind to two different antigens to be "bispecific" or bind to more than one different antigens to be "multi-specific". The antibody may be monovalent, bivalent or multivalent, i.e. the antibody may bind to one, two or multiple antigen molecules at one time. The antibody "monovalently" binds to a particular protein, that is, one molecular of an antibody binds to merely one molecular of a protein, but the antibody may also bind to different proteins. When the antibody binds to each of two different proteins, the antibody binds to each protein "monovalently" and the antibody is "bispecific" and "monovalently" binds to each of the two different proteins. The antibody may be "monomeric," that is, the antibody includes a single polypeptide chain. The antibody may include multiple polypeptide chains ("multimeric") or may include two ("dimeric"), three ("trimeric") or four ("tetrameric") polypeptide chains. If the antibody is multimeric, the antibody may be a homomultimer, that is, the antibody includes more than one molecular of only one type of polypeptide chain, including a homodimer, a homotrimer or a homotetramer. Alternatively, the multimeric antibody may be a heteromultimer, that is, the antibody includes more than one type of polypeptide chains that are different, including a heterodimer, a heterotrimer or a heterotetramer.

The term "monoclonal antibody (mAb)" refers to an antibody obtained from a population of substantially homogeneous antibodies, that is, individual antibodies comprised in the population are identical except for possible naturally occurring mutations that present in minor amounts. Therefore, the modifier "monoclonal" means that the property of the antibody is not a mixture of discrete antibodies. The monoclonal antibody is produced by methods known to those skilled in the art, such as the preparation of heterozygous antibody-producing cells through the fusion of myeloma cells and immune spleen cells. The monoclonal antibody can be synthesized by culturing hybridomas without being contaminated by any other immunoglobulins. The monoclonal antibody may be obtained through recombination technology, phage display technology, synthesis technology or other existing technologies.

The term "intact antibody" refers to an antibody consisting of two antibody heavy chains and two antibody light chains. An "intact antibody heavy chain" is composed of an antibody heavy chain variable domain (VH), antibody heavy chain constant domain 1 (CH1), an antibody hinge region (HR), antibody heavy chain constant domain 2 (CH2) and antibody heavy chain constant domain 3 (CH3) from N-terminus to C-terminus, abbreviated as VH-CH1-HR-CH2-CH3; and optionally includes antibody heavy chain constant domain 4 (CH4) in the case of an antibody of the IgE subclass. Preferably, the "intact antibody heavy chain" is a polypeptide consisting of VH, CH1, HR, CH2 and CH3 from the N-terminus to the C-terminus. An "intact antibody light chain" is a polypeptide consisting of an antibody light chain variable domain (VL) and an antibody light chain constant domain (CL) from N-terminus to C-terminus, abbreviated as VL-CL. The antibody light chain constant domain (CL) may be κ (kappa) or λ (lambda). Intact antibody chains are linked together by an inter-peptide disulfide bond between the CL domain and the CH1 domain (i.e., between the light chain and the heavy chain) and an inter-peptide disulfide bond between hinge regions of intact antibody heavy chains. Typical examples of the intact antibody are natural antibodies such as IgG (for example, IgG1 and IgG2), IgM, IgA, IgD and IgE.

The term "antibody fragment" or "antigen-binding fragment" refers to an antigen-binding fragment of an antibody that retains a specific binding ability to an antigen as well as antibody analogs and generally includes at least part of an antigen-binding region or a variable region of a parental antibody. The antibody fragment retains at least part of the binding specificity of the parental antibody. Generally, when activity is represented in moles (K_{D}), the antibody fragment retains at least 10% of parental binding activity. Preferably, the antibody fragment retains at least 20%, 50%, 70%, 80%, 90%, 95% or 100% of the binding affinity of the parental antibody to a target. Antibody fragments include, but are not limited to, Fab fragments, Fab' fragments, F(ab')₂ fragments, Fv fragments, Fd fragments, complementarity-determining region (CDR) fragments, disulfide-stabilized variable fragments (dsFv); linear antibodies, single-chain antibodies (for example, scFv monoclonal antibodies), unibodies (technology from Genmab), bivalent single-chain antibodies, single-chain phage antibodies, single domain antibodies (for example, VH domain antibodies), domain antibodies (technology from Domantis), nanobodies (technology from Ablynx); multi-specific antibodies formed from antibody fragments (for example, tribodies and tetrabodies); and engineered antibodies such as chimeric antibodies (for example, humanized murine antibodies) and heteroconjugate antibodies. These antibody fragments are obtained using any conventional technologies known to those skilled in the art, and the utility of these fragments is screened by the same method as the intact antibody.

The term "single-chain Fv antibody" (or "scFv antibody") refers to an antibody fragment comprising VH and VL domains of an antibody and is a fusion protein of a heavy chain variable region (VH) and a light chain variable region (VL) connected with a linker. The linker enables the two domains to be cross-linked to form an antigen-binding site, and the sequence of the linker generally consists of a flexible peptide, such as, but not limited to, G₂(GGGGS)₃. The size of scFv is generally 1/6 of an intact antibody. The single-chain antibody is preferably an amino acid chain sequence encoded by a nucleotide chain. For the review of scFv, reference may be made to Pluckthun A, 1994. Antibodies from Escherichia coli, in the Pharmacology of Monoclonal Antibodies, Vol 113, Rosenberg M and Moore GP (EDs.), Springer-Verlag, New York, pp 269-315. Reference may also be made to International Patent Application Publication No. WO 88/01649 and U.S. Patent Nos. 4946778 and 5260203.

The term "VL domain" refers to an amino-terminal variable domain of an immunoglobulin light chain.

The term "VH domain" refers to an amino-terminal variable domain of an immunoglobulin heavy chain.

The term "hinge region" includes a portion of a heavy chain molecule that links the CH1 domain to the CH2 domain. The hinge region includes about 25 residues and is flexible so that two N-terminal antigen-binding regions move independently. The hinge region may be divided into three different domains: upper, middle and lower hinge domains (Roux KH et al., 1998, J Immunol, 161: 4083-4090).

The term "Fab fragment" consists of a light chain and CH1 and a variable region of a heavy chain. The heavy chain of the Fab molecule cannot form a disulfide bond with another heavy chain molecule. The size of an "Fab antibody" is 1/3 of an intact antibody, and the "Fab antibody" includes only one antigen-binding site.

The term "Fab' fragment" contains a light chain, a VH domain and a CH1 domain of a heavy chain and a constant region between CH1 and CH2 domains.

The term "F(ab')₂ fragment" contains two light chains, VH domains and CH1 domains of two heavy chains and constant regions between CH1 and CH2 domains so that an inter-chain disulfide bond is formed between the two heavy chains. Therefore, the F(ab')₂ fragment is composed of two Fab' fragments held together by the disulfide bond between the two heavy chains.

The term "Fd fragment" consists of CH1 and a variable region of a heavy chain and is the heavy chain part of the Fab fragment excluding the light chain.

The term "Fv region" includes variable regions from the heavy chain and the light chain but lacks constant regions and is the minimum fragment containing a complete antigen recognition and binding site.

The term "disulfide-stabilized variable fragment (dsFv)" introduces one cysteine mutation site into the VH region and the VL region, respectively, so as to form a disulfide bond between the VH and the VL to achieve structural stability. The term "disulfide bond" includes a covalent bond formed between two sulfur atoms. The amino acid cysteine contains a sulfhydryl group which can form a disulfide bond or a bridge with a second sulfhydryl group. In most naturally occurring IgG molecules, CH1 and CK regions are linked by a disulfide bond and two heavy chains are linked by two disulfide bonds at positions 239 and 242 according to the Kabat numbering system (position 226 or 229, EU numbering system).

The term "heavy chain constant region" includes an amino acid sequence from an immunoglobulin heavy chain. A polypeptide comprising the heavy chain constant region includes at least one of: a CH1 domain, a hinge domain (for example, an upper hinge region, an intermediate hinge region and/or a lower hinge region), a CH2 domain, a CH3 domain or a variant or fragment thereof. For example, an antigen-binding polypeptide used in the present application may include a polypeptide chain having a CH1 domain; a polypeptide having a CH1 domain, at least part of a hinge domain and a CH2 domain; a polypeptide chain having a CH1 domain and a CH3 domain; a polypeptide chain having a CH1 domain, at least part of a hinge domain and a CH3 domain; or a polypeptide chain having a CH1 domain, at least part of a hinge domain, a CH2 domain and a CH3 domain. In another embodiment, the polypeptide of the present application includes a polypeptide chain having a CH3 domain. In addition, the antibody used in the present application may lack at least part of a CH2 domain (for example, all or part of the CH2 domain). As described above, it is appreciated by those of ordinary skill in the art that heavy chain constant regions may be modified such that they differ in amino acid sequence from naturally occurring immunoglobulin molecules.

The term "light chain constant region" includes an amino acid sequence from an antibody light chain. Preferably, the light chain constant region includes at least one of a constant kappa domain and a constant lambda domain.

The term "Fc region" or "Fc fragment" refers to a C-terminal region of an immunoglobulin heavy chain, which includes at least part of a hinge region, a CH2 domain and a CH3 domain. It mediates the binding of immunoglobulins to host tissues or factors, including the binding of immunoglobulins to Fc receptors located on various cells (for example, effector cells) of an immune system or the binding of immunoglobulins to the first component (C1q) of a classical complement system. The Fc region includes a native sequence Fc region and a variant Fc region.

Generally, a human IgG heavy chain Fc region is a segment from an amino acid residue at position Cys226 or Pro230 of the human IgG heavy chain Fc region to the carboxy terminus, but its boundary may vary. The C-terminal lysine of the Fc region (residue 447, according to the EU numbering system) may or may not be present. Fc may also refer to this region in isolation or in the case of a protein polypeptide comprising Fc, for example, a "binding protein comprising the Fc region", which is also referred to as "Fc fusion protein" (for example, antibody or immunoadhesin). The native sequence Fc region of the antibody of the present application includes mammalian (for example, human) IgG1, IgG2 (IgG2A or IgG2B), IgG3 and IgG4. In some embodiments, there is a single amino acid substitution, insertion and/or deletion of about 10 amino acids per 100 amino acids in the amino acid sequences of two Fc polypeptide chains relative to the amino acid sequence of the mammalian Fc polypeptide. In some embodiments, the amino acid difference in the Fc region may be Fc changes that extend a half-life, changes that increase FcRn binding, changes that enhance Fcγ receptor (FcyR) binding and/or changes that enhance ADCC, ADCP and/or CDC.

In antibody isotypes IgG, IgA and IgD, the Fc region includes CH2 and CH3 constant domains of each of two heavy chains of the antibody; the Fc region of IgM and IgE includes three heavy chain constant domains (CH2 to CH4 domains) in each polypeptide chain.

The term "chimeric antibody" refers to an antibody in which a portion of a heavy chain and/or a light chain is identical or homologous to a corresponding sequence in an antibody derived from a particular species or belonging to a particular antibody class or subclass, while the remainder of the chain is identical or homologous to a corresponding sequence in an antibody derived from another species or belonging to another antibody class or subclass, and a fragment of such antibody, as long as they exhibit desired biological activity (U.S. patent No. 4816567; Morrison SL et al., 1984, Proc Natl Acad Sci USA, 81: 6851-6855). For example, the term "chimeric antibody" may include such an antibody (such as a human-murine chimeric antibody) in which the heavy and light chain variable regions of the antibody are from a first antibody (such as a murine antibody) and the heavy and light chain constant regions of the antibody are from a second antibody (such as a human antibody).

The term "humanized antibody" refers to a genetically engineered non-human antibody whose amino acid sequence has been modified to increase homology to the sequence of a human antibody. Most or all of amino acids outside CDR domains of the non-human antibody, for example, a mouse antibody, are substituted with corresponding amino acids from human immunoglobulins, while most or all of amino acids in one or more CDR regions are not altered. The addition, deletion, insertion, substitution or modification of amino acids is permissible as long as they do not eliminate the ability of the antibody to bind to a particular antigen. The "humanized" antibody retains antigen specificity similar to that of the original antibody. The origin of the CDRs is not particularly limited and may be derived from any animal. For example, CDR regions derived from mouse antibodies, rat antibodies, rabbit antibodies or non-human primate (for example, cynomolgus monkeys) antibodies may be used. For a framework region, human antibody germline sequences can be obtained by searching IMGT antibody germline database (http://www.imgt.org/3Dstructure-DB/cgi/DomainGapAlign.cgi). Generally, a human antibody germline sequence with high homology to the modified non-human antibody is selected as the framework region of the humanized antibody.

The term "hypervariable region", "CDR" or "complementarity-determining region" refers to amino acid residues of an antibody, which are responsible for antigen binding, and is a discontinuous amino acid sequence. A CDR sequence includes amino acid residues in a variable region that may be defined by the Kabat, Chothia, IMGT (Lefranc et al, 2003, Dev Comparat Immunol, 27: 55-77) or AbM (Martin ACR et al, 1989, Proc Natl Acad Sci USA, 86: 9268-9272) method or identified by any CDR sequence determination method well known in the art. For example, the hypervariable region includes the following amino acid residues: amino acid residues from a "complementarity-determining region" or "CDR" defined by sequence comparison (Kabat numbering system), for example, residues at positions 24-34 (CDR-L1), 50-56 (CDR-L2) and 89-97 (CDR-L3) in a light chain variable domain and residues at positions 31-35 (CDR-H1), 50-65 (CDR-H2) and 95-102 (CDR-H3) in a heavy chain variable domain (see Kabat et al, 1991, Sequences of Proteins of Immunological Interest, 5th Edition, Public Health Service, National Institutes of Health, Bethesda, Md.); and/or residues from a "hypervariable loop" (HVL) defined according to the structure (Chothia numbering system), for example, residues at positions 26-32 (CDR-L1), 50-52 (CDR-L2) and 91-96 (CDR-L3) in the light chain variable domain and residues at positions 26-32 (CDR-H1), 53-55 (CDR-H2) and 96-101 (CDR-H3) in the heavy chain variable domain (see Chothia C and Lesk AM, 1987, J Mol Biol, 196: 901-917; Chothia C et al, 1989, Nature, 342: 878-883). "Framework" residues or "FR" residues refer to variable domain residues other than the residues of the hypervariable region as defined herein. In some embodiments, the CDRs contained in the antibody or the antigen-binding fragment thereof in the present application are preferably determined through the Kabat, IMGT or Chothia numbering system. Those skilled in the art may explicitly assign each numbering system to any variable domain sequence without relying on any experimental data beyond the sequence itself. For example, the Kabat residue numbering method of a given antibody may be determined by comparing the sequence of the given antibody to each "standard" numbered sequence. Based on the sequence numbering schemes provided herein, the determination of any variable region sequences in the sequence table is entirely within the conventional technical scope of those skilled in the art.

The term "recombinant" refers to polypeptides or polynucleotides refers to a form of polypeptides or polynucleotides that is not present in a natural state. As one non-limiting example, it may be achieved through a combination of polynucleotides or polypeptides that are generally not present together.

The term "vector" refers to a nucleic acid molecule capable of transporting another nucleic acid to which it is linked. One type of vector is a "plasmid", which refers to a circular double-stranded DNA loop into which an additional DNA segment can be linked. Another type of vector is a viral vector in which an additional DNA segment may be linked into a viral genome. Some vectors can be self-replicated in host cells into which these vectors are introduced (for example, a bacterial vector with a bacterial replication origin and an episomal mammalian vector). Other vectors (such as non-additional mammalian vectors) may be integrated into the genome of a host cell after being introduced into the host cell and thus replicated with the genome of the host cell. In addition, some vectors can direct the expression of genes to which they are effectively linked. Such vectors are referred to as "recombinant expression vectors" (or simply referred to as "expression vectors") herein. In general, expression vectors useful in recombinant DNA techniques are generally present in the form of plasmids. However, other forms of expression vectors are also included, such as viral vectors (for example, replication-defective retroviruses, adenoviruses and adeno concomitant viruses), which perform equivalent functions.

The term "isolated antibody molecules" refers to antibody molecules that have been identified and isolated and/or recovered from components in their natural environment. Contaminant components in the natural environment of the antibody molecules are substances that interfere with the diagnostic or therapeutic use of the antibody and may include enzymes, hormones and other proteinaceous or non-proteinaceous solutes.

The term "isolated" related to nucleic acids (such as DNA or RNA) means that the molecule is isolated from other DNA or RNA which is present as macromolecules of natural origin. The term "isolated" used herein also refers to nucleic acids or peptides that, when manufactured by the recombinant DNA technology, are substantially free of cellular materials, viral materials or culture media or those that are substantially free of chemical precursors or other chemicals when prepared by chemical synthesis. In addition, "isolated nucleic acids" refer to nucleic acid fragments that are not naturally generated as fragments and are not present in the natural state. The term "isolated" used herein also refers to cells or polypeptides isolated from other cellular proteins or tissues. Isolated polypeptides include purified and recombinant polypeptides.

The term "cross-reaction" refers to the ability of the antibody described herein to bind to antigens from different species. Cross-reactivity may be measured by detecting specific reactivity with purified antigens in binding assays (for example, SPR and ELISA) or by detecting the binding to cells physiologically expressing antigens or the interaction with the function of cells physiologically expressing antigens. Examples of assays known in the art for determining the binding affinity include surface plasmon resonance (for example, Biacore) or similar techniques (for example, Kinexa or Octet).

The term "immunobinding" or "immunobinding property" refers to a non-covalent interaction between an immunoglobulin molecule and an antigen (to the antigen, the immunoglobulin is specific). The strength or affinity of an immunobinding interaction may be represented by the equilibrium dissociation constant (K_{D}) of the interaction, wherein the smaller the K_{D} value, the higher the affinity. The immunobinding property of the selected polypeptide may be determined by a method known in the art. One determination method relates to the measurement of a rate at which an antigen/antibody complex is formed and dissociated. Both the "binding rate constant" (Kₐ or Kₒₙ) and the "dissociation rate constant" (K_{d} or K_{off}) may be calculated according to the concentration and actual rates of association and dissociation (see Malmqvist M, 1993, Nature, 361: 186-187). The ratio of k_{d}/kₐ is the equilibrium dissociation constant K_{D} (see Davies DR et al, 1990, Annual Rev Biochem, 59: 439-473). Any effective method may be used for measuring values of K_{D}, kₐ and k_{d}.

The term "immunogenicity" refers to an ability of a particular substance to elicit an immune response.

The term "host cell" refers to a cell in which a vector can proliferate and its DNA can be expressed. The cell may be a prokaryotic or eukaryotic cell. The term also includes any offspring of the test host cell. It is to be understood that not all offspring are the same as parent cells due to mutations during replication and such offspring are included. The host cell includes a prokaryotic cell, a yeast cell or a mammalian cell such as a CHO cell, a NS0 cell or another mammalian cell.

The term "identity" refers to the matching of sequences between two polypeptides or between two nucleic acids. When a certain position in each of two sequences for comparison is occupied by the same base group or amino acid monomer subunit (for example, a certain position in each of two DNA molecules is occupied by adenine or a certain position in each of two polypeptides is occupied by lysine), then the molecules are identical at the position. The "percentage identity" between two sequences is a function of the number of matching positions of the two sequences divided by the number of positions to be compared and then multiplied by 100. For example, if 6 positions of 10 positions in two sequences are matched, the two sequences have 60% identity. For example, DNA sequences CTGACT and CAGGTT have a total identity of 50% (three positions of a total of six positions are matched). Generally, the comparison is made when two sequences are aligned to produce maximum identity. Such alignment may be conveniently implemented through a computer program, such as an Align program (DNAstar, Inc.), by the method described by Needleman and Wunsch (Needleman SB and Wunsch CD, 1970, J Mol Biol, 48: 443-453).

The term "mutated", "mutant" or "mutation" refers to the substitution, deletion or insertion of one or more nucleotides or amino acids relative to natural nucleic acids or polypeptides (i.e., reference sequences that may be used for defining wild types).

The term "conservative modification" is intended to mean that an amino acid modification does not significantly affect or change the binding characteristics of the antibody containing an amino acid sequence. Such conservative modifications include amino acid substitutions, additions and deletions. A modification may be introduced into the antibody of the present application by using a standard technology known in the art, such as a site-directed mutation and a PCR-mediated mutation. A conservative amino acid substitution refers to the substitution of an amino acid residue with an amino acid residue with a similar side chain. Families of amino acid residues with similar side chains have been described in detail in the art. These families include amino acids with basic side chains (such as lysine, arginine and histidine), amino acids with acidic side chains (such as aspartic acid and glutamic acid), amino acids with uncharged polar side chains (such as glycine, asparagine, glutamine, serine, threonine, tyrosine, cysteine and tryptophan), amino acids with non-polar side chains (such as alanine, valine, leucine, isoleucine, proline, phenylalanine and methionine), amino acids with β-branched side chains (such as threonine, valine and isoleucine) and amino acids with aromatic side chains (such as tyrosine, phenylalanine, tryptophan and histidine). Therefore, one or more amino acid residues in the CDR of the antibody of the present application may be substituted with other amino acid residues from the same side chain family.

The antibody of the present application or a nucleic acid or polynucleotide encoding the antibody of the present application may be applied to the preparation of a pharmaceutical composition or a sterile composition. For example, the antibody is mixed with a pharmaceutically acceptable carrier, excipient or stabilizer. The pharmaceutical composition may include one antibody or a combination of (for example, two or more different) antibodies of the present application. For example, the pharmaceutical composition of the present application may include a combination of antibodies or antibody fragments (or immunoconjugates) that have complementary activity and bind to different epitopes on a target antigen. Formulations of therapeutic and diagnostic agents may be prepared by mixing the antibody with the pharmaceutically acceptable carrier, excipient or stabilizer in the form of, for example, lyophilized powder, slurry, an aqueous solution or a suspension. The term "pharmaceutically acceptable" means that a molecule, molecule fragment or composition does not produce unfavorable, allergic or other adverse effects when properly administered to animals or humans. Specific examples of some substances that may act as the pharmaceutically acceptable carriers or components thereof include sugars (such as lactose), starch, cellulose and its derivatives, vegetable oils, gelatin, polyols (such as propylene glycol) and alginic acid. The antibody of the present application or the nucleic acid or polynucleotide encoding the antibody of the present application may be used alone or in combination with one or more other therapeutic agents such as vaccines.

The term "pharmaceutically acceptable carrier and/or excipient and/or stabilizer" refers to a carrier and/or excipient and/or stabilizer that are pharmacologically and/or physiologically compatible with a subject and an active ingredient and that are non-toxic to cells or mammals exposed to the carrier and/or excipient and/or stabilizer at the dose and concentration used. The pharmaceutically acceptable carrier and/or excipient and/or stabilizer include, but are not limited to, a PH regulator, a surfactant, an adjuvant, an ionic strength enhancer, a diluent, a reagent for maintaining osmotic pressure, a reagent for delaying absorption and a preservative. For example, the pH regulator includes, but is not limited to, a phosphate buffer. The surfactant includes, but is not limited to, cationic, anionic or nonionic surfactants, such as Tween-80. The ionic strength enhancer includes, but is not limited to, sodium chloride. The preservative includes, but is not limited to, various antibacterial reagents and antifungal reagents such as p-hydroxybenzoate, chloretone, phenol and sorbic acid. The reagent for maintaining osmotic pressure includes, but is not limited to, sugar, NaCl and analogues thereof. The reagent for delaying absorption includes, but is not limited to, monostearate and gelatin. The diluent includes, but is not limited to, water, an aqueous buffer (such as buffered saline), an alcohol and a polyol (such as glycerol). The preservative includes, but is not limited to, various antibacterial reagents and antifungal reagents such as thimerosal, 2-phenoxyethanol, p-hydroxybenzoate, chloretone, phenol and sorbic acid. The stabilizer has the meaning generally understood by those skilled in the art, can stabilize the desired activity of active ingredients in a drug, and includes, but is not limited to, sodium glutamate, gelatin, SPGA, sugars (for example, sorbitol, mannitol, starch, sucrose, lactose, dextran or glucose), amino acids (for example, glutamate, glycine), proteins (for example, dried whey, albumin or casein) or degradation products thereof (for example, lactalbumin hydrolysate).

As used herein, the term "effective amount" refers to an amount sufficient to obtain or at least partially obtain the desired effect. For example, a prophylactically (for example, tumor, infection or autoimmune disease) effective amount refers to an amount sufficient to prevent, arrest or delay the onset of a disease (for example, tumor, infection or autoimmune disease); a therapeutically effective amount refers to an amount sufficient to cure or at least partially arrest the disease and complications thereof in a patient already suffering from the disease. It is well within the ability of those skilled in the art to determine such effective amounts. For example, the amount effective for therapeutic use depends on the severity of the to-be-treated disease, the overall state of the patient's immune system, the general condition of the patient such as age, weight and sex, the mode of administration of the drug, and other treatments administered concurrently.

As used herein, the term "immune cells" include cells of hematopoietic origin that function in immune responses, for example, lymphocytes such as B cells and T cells; natural killer cells; and myeloid cells such as monocytes, macrophages, eosinophils, mast cells, basophils and granulocytes.

As used herein, the term "immune response" refers to the action of immune cells (such as lymphocytes, antigen-presenting cells, macrophages or granulocytes) and soluble macromolecules produced by the immune cells or the liver (including antibodies, cytokines and complements) that results in selective injuries to, damages to or elimination from the human body of invading pathogens, cells or tissues infected with pathogens, cancer cells or normal human cells or tissues in the context of autoimmunization or pathological inflammation. In the present application, the term "antigen-specific T cell response" refers to an immune response produced by T cells that occurs when an antigen specific to the T cells stimulates the T cells. Non-limiting examples of a reaction produced by the T cells upon antigen-specific stimulation include the proliferation of T cells and the production of cytokines (such as IL-2).

As used herein, the term "effector function" refers to biological activities that can be attributed to the biological activities of the antibody Fc region (a native sequence Fc region or amino acid sequence variant Fc region) and that vary with antibody isotypes. Examples of antibody effector functions include, but are not limited to, Fc receptor binding affinity, antibody-dependent cell-mediated cytotoxicity (ADCC), complement-dependent cytotoxicity (CDC), antibody-dependent cell-mediated phagocytosis (ADCP), downregulation of cell surface receptors (for example, B cell receptors), B cell activation, cytokine secretion and half-life/clearance rate of antibodies and antigen-antibody complexes. Methods of altering the antibody effector functions are known in the art, for example, the effector function may be altered by introducing mutations in the Fc region.

As used herein, the term "antibody-dependent cell-mediated cytotoxicity (ADCC)" refers to a cytotoxic form in which Ig binds to FcRs on cytotoxic cells (for example, natural killer (NK) cells, neutrophils or macrophages) to enable these cytotoxic effector cells to specifically bind to antigen-attached target cells and then secret cytotoxins to kill the target cells. Methods of detecting the ADCC activity of an antibody are known in the art, for example, the ADCC activity may be detected by measuring the binding activity between a to-be-tested antibody and FcR (for example, CD16a).

As used herein, the term "complement-dependent cytotoxicity (CDC)" refers to a cytotoxic form that activates the complement cascade by binding the complement component C1q to the antibody Fc. Methods of detecting the CDC activity of an antibody are known in the art, for example, the CDC activity may be detected by measuring the binding activity between a to-be-tested antibody and FcR (for example, C1q).

Embodiments of the present application are further described by the examples given below. It is to be understood by those skilled in the art that the following drawings and examples are illustrative of the present application and are not intended to further limit the present application.

Compared with the existing art, technical solutions of the present application have the beneficial effects below.
(1) The antibody of the present application can not only specifically recognize/bind to CD3 but also induce antigen regulation, including an alteration (for example, reduction) of cell surface expression levels or an alteration of CD3 or TCR activity. Therefore, the antibody of the present application has potential for preventing and/or treating the tumor, organ transplantation rejection or autoimmune disease.
(2) The antibody (particularly humanized antibody) of the present application not only retains the function and property of a parental murine antibody to have the potential for preventing and treating the tumor, infection or autoimmune disease but also has a very high degree of humanization to be safely administered to a human subject without eliciting an immunogenic response. Therefore, the antibody (particularly humanized antibody) of the present application has great clinical values.
(3) The antibody of the present application can significantly promote T cell proliferation and activate T cells and control a degree of cell activation (release of cytokines such as IL-2) to be within a safe and effective range without eliciting the excessive release of cytokines, thus having higher safety and reducing toxic side effects in clinical therapy.

### BRIEF DESCRIPTION OF DRAWINGS

FIG. 1 shows the determination of abilities of murine antibodies AP191, AP591 and AP831 to bind to an antigen, human CD3.
FIG. 2 shows the determination of activities of murine antibodies AP191, AP591 and AP831 to bind to human primary T cells.
FIG. 3 shows the detection of abilities of murine antibodies AP191, AP591 and AP831 to promote PBMC cell proliferation.
FIG. 4 shows the determination of abilities of humanized antibodies AB611 and AB614 to bind to antigens, human CD3 and cynomolgus monkey CD3.
FIG. 5 shows the determination of activities of humanized antibodies AB611 and AB614 to bind to human primary T cells.
FIG. 6 shows the detection of abilities of humanized antibodies AB610, AB611 and AB614 to promote PBMC cell proliferation.
FIG. 7 shows the evaluation of an ability of a humanized antibody AB614 to activate T cells.

### DETAILED DESCRIPTION

The present application is described with reference to the following examples, which are intended to illustrate the present application (instead of limiting the present application).

Unless otherwise specified, experimental methods in molecular biology and immunoassays used in the present application are substantially performed with reference to J. Sambrook et al., Molecular Cloning: A Laboratory Manual, 2nd edition, Cold Spring Harbor Laboratory, 1989, and F. M. Ausubel et al., Short Protocols in Molecular Biology, 3rd edition, John Wiley & Sons, Inc., 1995; and the use of restriction enzymes is in accordance with conditions recommended by the product manufacturer. It is known to by those skilled in the art that the examples illustrate the present application by way of example and are not intended to limit the scope of the present application as claimed.

### Example 1 Preparation of a murine monoclonal antibody against human CD3

50 µg/mouse of human CD3E antigen (protein sequence: Uniprot entry No. P07766) was fully emulsified with complete Freund's adjuvant and immunized male Balb/C mice by a multi-site immunization method once every three weeks. On the 10th day after the third immunization, blood was sampled from the caudal vein, the titer of anti-human CD3 antibody in plasma was tested by ELISA to monitor the degree of immune response in mice, and then a mouse with the highest titer of anti-human CD3 antibody was boosted once 3 days before fusion. Three days later, the mouse was sacrificed and its spleen was removed and fused with a mouse Sp2/0 myeloma cell strain. 2×10⁸ Sp2/0 cells were fused with 2×10⁸ spleen cells in a solution of 50% polyethylene glycol (with a molecular weight of 1450) and 5% dimethyl sulfoxide (DMSO). The number of spleen cells was adjusted to 5×10⁵/mL by using Iscove's medium (containing 10% fetal bovine serum, 100 U/mL penicillin, 100 µg/mL streptomycin, 0.1 mM hypoxanthine, 0.4 µM aminopterin and 16 µg thymidine), and 0.3 mL was added to the wells of a 96-well plate and placed in a 37°C, 5% CO₂ incubator. After 10 days of culture, clones where antibodies in the supernatant bind to CD3E with high affinity were detected separately by high-throughput ELISA. Then the fused cells in the wells of the above monoclonal antibodies were subcloned and further screened to obtain hybridoma cell strains #191, #591 and #831.

A clone that produced a specific antibody was cultured in RPMI 1640 medium supplemented with 10% FCS. When the cell density reaches approximately 5×10⁵ cells/mL, the medium was replaced with a serum-free medium. After 2 to 4 days, the cultured medium was centrifuged to collect the culture supernatant. The antibody was purified with protein G column. The eluent of the monoclonal antibody was dialyzed against 150 mM NaCl. The dialyzed solution was filtered and sterilized through a 0.2 µm filter to obtain the purified murine monoclonal antibodies AP191, AP591 and AP831 to be tested.

### Example 2 Determination of abilities of murine antibodies to bind to human CD3 antigen by ELISA

A microtiter plate was coated with 100 µL of 0.1 µg/mL human CD3E (purchased from Acro Biosystems) overnight at room temperature. The coating solution was discarded, and the wells were blocked with skimmed milk dissolved in phosphate buffered saline (PBS) for 0.5 hours and washed with PBS containing 0.05% Tween-20 (PBST). Then the purified murine antibodies AP191, AP591 and AP831 against human CD3 were added with 50 µL per well and incubated for 1 h at room temperature. The wells were washed with 0.05% PBST. Then 50 µL of HRP-labeled goat anti-mouse IgG polyclonal antibody (purchased from Jackson Laboratory) was added per well as the detected antibody and incubated for 1 h at 37 °C. The wells were washed three times with 0.05% PBST, added with TMB (100 µL/well) and developed at room temperature for 5 min. 0.2 M H₂SO₄ was added to terminate the reaction, 50 µL/well. Absorption values were read by a microplate reader at double wavelengths 450 nm/620 nm. Plotting was performed by software GraphPad Prism 6 with OD 450 nm/620 nm as the Y-axis and an antibody concentration as the X-axis.

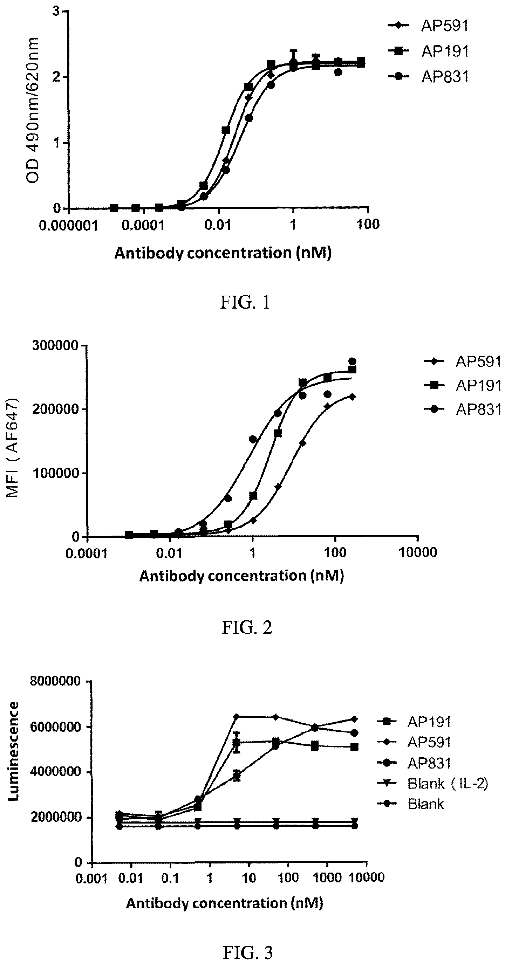

As shown in FIG. 1, the murine antibodies AP191, AP591 and AP831 all had relatively high affinity to human CD3E, and the EC₅₀ values of AP191, AP591 and AP831 binding to human CD3E molecules were 0.01491 nM, 0.02826 nM and 0.04038 nM, respectively.

### Example 3 Affinity determination and kinetic study of CD3 murine antibodies

The binding affinity constants of the purified murine monoclonal antibodies to antigen CD3E were determined by biofilm interferometry (BLI) with the ForteBio Octet RED&QK system from PALL Corporation. Concentration gradients of a multichannel parallel quantitative analysis were set to 3.125, 6.25, 12.5, 25, 50 and 100 nM, and His-labeled human CD3E (10 µg/mL) was coupled to Ni-NTA sensors. The affinity determination results are shown in Table 1. The results show that the murine monoclonal antibodies have very high binding affinity to human CD3, which can be on the order of 10⁻¹⁰ M.

**Table 1 Affinity determination results of murine monoclonal antibodies**

| Antibody | K_{D} (M) | Ka (1/Ms) | Kd (1/s) |
|---|---|---|---|
| AP191 | 1.210E-10 | 8.020E+05 | 9.730E-05 |
| AP591 | 1.937E-11 | 5.390E+05 | 1.044E-05 |
| AP831 | 2.333E-10 | 6.922E+05 | 1.615E-05 |

### Example 4 Evaluation of in vitro biological functions of CD3 murine antibodies

### 4.1. Activities of the CD3 murine antibodies to bind to human primary T cells

The human primary T cells were cultured and centrifuged to collect cells, the cells were resuspended in a PBS solution containing 1% BSA (PBSB), the cell density was adjusted to 1×10⁶ cells/mL, and the cells were added to a 96-well U plate (100 µL/well) and blocked for 30 min at 4 °C. The cells were washed once with 1% PBSB. The diluted CD3 murine antibodies AP591, AP191 and AP831 with a series of concentrations were added (100 µL/well) and incubated for 1 h at 4 °C. The wells were centrifuged to remove the supernatant and washed twice with 1% PBSB, and the diluted goat anti-mouse IgG (H+L) antibody AF647 (Jackson Immuno Research Inc., diluted at 1:250) was added (100 µL/well) and incubated for 1 h at 4 °C in the dark. The wells were centrifuged to remove the supernatant and washed twice with 1% PBSB, and 4% paraformaldehyde (PFA) was added (200 µL/well) to resuspend the cells, and signal strength was detected by flow cytometry. With the mean fluorescence intensity as the Y-axis and the antibody concentration as the X-axis, the EC₅₀ values of the CD3 murine antibodies binding to the human primary T cells were analyzed and calculated by software GraphPad Prism 6.

As shown in FIG. 2, at the cellular level, the CD3 murine antibodies bind to the human primary T cells, the signal strength is directly proportional to the antibody concentration, the binding effects are AP831 > AP191 > AP591, and the EC₅₀ values of AP591, AP191 and AP831 binding to the human primary T cells are 8.907 nM, 2.743 nM and 0.825 nM, respectively.

### 4.2 Detection of abilities of CD3 murine antibodies to promote PBMC cell proliferation

A cell viability detection kit by CellTiter-Glo^{®} luminescence is a method of detecting the number of living cells in the culture by quantitatively determining ATP.

Fresh PBMCs were obtained from human peripheral blood through density gradient centrifugation and frozen in liquid nitrogen for storage and use. The frozen PBMCs were resuscitated, the cell density was adjusted to 5×10⁵ cells/mL, 100 µL/well in a 1640 medium containing 10% FBS, and the cells were added to a 96-well plate. The CD3 murine antibodies AP591, AP191 and AP831 were diluted to 10 µg/mL in a medium, 10-fold dilution into 7 gradients, 100 µL/well was added to the 96-well plate with two duplicate wells set, and they were incubated for 20 h in a 37 °C, 5% CO₂ incubator. 1000 IU/mL IL-2 was added (50 µL/well) to the 96-well plate and cultured for 48 h in the 37 °C, 5% CO₂ incubator. A control group with no antibody and no IL-2 (Blank) and a control group with no antibody and only IL-2 (Blank (IL-2)) were also set. The cells in the 96-well plate were blown uniformly, 100 µL of cell suspension was pipetted into a 96-well white plate, 100 µL of CellTiter-Glo^{®} luminescence cell viability detection reagent (Promega, G7571) was added, and the cells were shaken in a shaker and incubated for 10 min. Cold luminescence values were detected with a microplate reader. A plotting analysis was performed by software GraphPad Prism 6 with the luminescence as the Y-axis and the antibody concentration as the X-axis.

As shown in FIG. 3, the CD3 murine antibodies AP591, AP191 and AP831 can all promote T cell proliferation, and AP591 has the best proliferation effect, followed by AP191 and AP831.

### Example 5 Subtype identification and variable region amplification of murine antibodies against human CD3

The culture supernatant of hybridoma cells was taken and the antibody subtype was identified using IsoStrip^{™} mouse monoclonal antibody subtype identification kit (from Santa Cruz Biotechnology). The subtype of the murine antibody AP591 was identified as IgG1 (Lambda), the subtype of AP191 was identified as IgG1 (Kappa), and the subtype of AP831 was identified as IgG1 (Kappa).

Amplification of variable regions of an antibody: a candidate hybridoma cell strain was cultured to 10⁷ cells in total and centrifuged at 1000 rpm for 10 min to collect cells. The total RNA was extracted with Trizol kit (Invitrogen), and the first strand of cDNA was synthesized with SMARTer RACE reverse transcription kit and used as a template for the subsequent amplification of DNA sequences of the variable regions of the antibody corresponding to hybridoma cells. According to the subtype identification result, the heavy and light chain constant region sequences of the antibody subtype were acquired, and specific nested PCR primers were designed, where the primer sequences used in the amplification were complementary to the first framework region of the variable region and the constant region of the antibody. A target gene was amplified by a conventional PCR method, and the amplified product was sequenced to obtain heavy chain variable region sequence SEQ ID NO: 1 and light chain variable region sequence SEQ ID NO: 2 of the antibody AP191 secreted by hybridoma cell strain #191, heavy chain variable region sequence SEQ ID NO: 3 and light chain variable region sequence SEQ ID NO: 4 of the antibody AP591 secreted by hybridoma cell strain #591, and heavy chain variable region sequence SEQ ID NO: 5 and light chain variable region sequence SEQ ID NO: 6 of the antibody AP831 secreted by hybridoma cell strain #831. The amino acid sequences of heavy chain CDRs (CDR-H1, CDR-H2 and CDR-H3) and light chain CDRs (CDR-L1, CDR-L2 and CDR-L3) of the above antibodies are shown in Tables 2-1 to 2-3, respectively.

**Table 2-1 Heavy and light chain CDRs of the murine antibody AP191**

| | **Kabat** | **IMGT** |
|---|---|---|
| CDR-H1 | SYYIH (SEQ ID NO: 7) | GYTFTSYY (SEQ ID NO: 13) |
| CDR-H2 | WIYPGDGSTKFNEKFRG (SEQ ID NO: 8) | IYPGDGST (SEQ ID NO: 14) |
| CDR-H3 | DGYSLYYFEF (SEQ ID NO: 9) | ARDGYSLYYFEF (SEQ ID NO: 15) |
| CDR-L1 | KSSQSLLNSRTRKTYLA (SEQ ID NO: 10) | QSLLNSRTRKTY (SEQ ID NO: 16) |
| CDR-L2 | WASTRDS (SEQ ID NO: 11) | WAS (SEQ ID NO: 17) |
| CDR-L3 | VQSYTLRT (SEQ ID NO: 12) | VQSYTLRT (SEQ ID NO: 12) |

**Table 2-2 Heavy and light chain CDRs of the murine antibody AP591**

| | **Kabat** | **IMGT** |
|---|---|---|
| CDR-H1 | TYAMN (SEQ ID NO: 18) | GFTFNTYA (SEQ ID NO: 24) |
| CDR-H2 | RIRSKINNYATYYADSVRD (SEQ ID NO: 19) | IRSKINNYAT (SEQ ID NO: 25) |
| CDR-H3 | HDNFYGSTYSWFAD (SEQ ID NO: 20) | VRHDNFYGSTYSWFAD (SEQ ID NO: 26) |
| CDR-L1 | RSSTGVVTTSNYAN (SEQ ID NO: 21) | TGVVTTSNY (SEQ ID NO: 27) |
| CDR-L2 | ATNYRVP (SEQ ID NO: 22) | ATN (SEQ ID NO: 28) |
| CDR-L3 | VLWYSNHWV (SEQ ID NO: 23) | VLWYSNHWV (SEQ ID NO: 23) |

**Table 2-3 Heavy and light chain CDRs of the murine antibody AP831**

| | **Kabat** | **IMGT** |
|---|---|---|
| CDR-H1 | NNYIH (SEQ ID NO: 29) | GYTFTNNY (SEQ ID NO: 35) |
| CDR-H2 | WIYPGDGTTKYNEKFKG (SEQ ID NO: 30) | IYPGDGTT (SEQ ID NO: 36) |
| CDR-H3 | NNNYYFDY (SEQ ID NO: 31) | TRNNNYYFDY (SEQ ID NO: 37) |
| CDR-L1 | KSSQSLFNSRTRKNYLA (SEQ ID NO: 32) | QSLFNSRTRKNY (SEQ ID NO: 38) |
| CDR-L2 | WASTRES (SEQ ID NO: 33) | WAS (SEQ ID NO: 39) |
| CDR-L3 | KQSFILRT (SEQ ID NO: 34) | KQSFILRT (SEQ ID NO: 34) |

The above CDR sequences are defined by the Kabat and IMGT methods separately, and any other CDR sequence determination method known in the art may also be used to identify amino acid residues in the CDRs in the variable regions.

### Example 6 Humanization of murine antibodies against human CD3

CDR grafting was used for the humanization of the murine antibodies. The basic principle of the CDR grafting is to graft the CDRs of a murine antibody onto a human antibody template and simultaneously introduce several or some key FR region residues of the murine antibody that stabilize the CDR conformation and that are important to antigen-antibody binding into the human antibody template (backmutations), so as to reduce the immunogenicity of the murine antibody and maintain the affinity of the murine antibody. Besides the above CDR grafting operation, the isoelectric point (PI), hydrophobic aggregation, post-translational modification (PTM) such as glycosylation, cleavage and isomerization sites and immunogenicity of the humanized antibody after the CDR grafting are further calculated, and amino acids causing problems in these four aspects are mutated so that the humanized antibody exerts full efficacy at the time of clinical use.

A specific flow of antibody humanization is described below. The IMGT human antibody germline database (http://www.imgt.org/3Dstructure-DB/cgi/DomainGapAlign.cgi) was searched to obtain a human antibody template with high similarity to the murine antibodies. The CDRs of the murine antibodies and the human antibody template were annotated with Discovery Studio to define the CDRs according to the Kabat or IMGT scheme. The six CDRs of the human antibody template were replaced with the six CDRs of the murine antibody, respectively. Each CDR of the six CDRs grafted may be an amino acid region defined by Kabat or an amino acid region defined by IMGT. After the CDR grafting, backmutations from the murine antibody to the FR region of the humanization template were performed. Key FR region amino acids of the murine antibody that stabilize the CDR conformation of the antibody and that are important to antigen-antibody binding include four classes of amino acid residues: (1) amino acids in the CDRs, within 6Å and hidden below the surface of the antibody; (2) amino acids in the CDRs, within 6Å and exposed on the surface of the antibody; (3) interfacial amino acids between light and heavy chain domains of the antibody; and (4) vernier zone residues that stabilize the CDR conformation of the antibody (Foote J and Winter G, 1992, J Mol Biol, 224: 487-499). The above four classes of key FR region residues of the murine antibody are determined by establishing a three-dimensional structural model of the murine antibody. As for the four classes of amino acids of the human template that are inconsistent with the sequences of the murine antibody, the three-dimensional structure was analyzed, amino acids important for maintaining the CDR conformation and the antigen-antibody binding were selected, and the grafting or substitution of the amino acids from the murine antibody to the human template was performed. Then, the isoelectric point, hydrophobic aggregation, post-translational modification and immunogenicity of the humanized antibody produced after the grafting of the four classes of amino acids were further calculated, and problematic amino acids were mutated to obtain the final humanized antibody sequence.

18 humanized antibodies were constructed based on the CDRs of the murine antibodies AP191, AP591 and AP831 by the above method, which were named AB610 to AB627, respectively. The amino acid sequences of the variable regions of the humanized antibodies are shown in Table 3.

**Table 3 amino acid sequences of the variable regions of the humanized antibodies**

| Antibody No. | Amino acid sequence of the heavy chain variable region | Amino acid sequence of the light chain variable region |
|---|---|---|
| **AB610** | SEQ ID NO: 40 | SEQ ID NO: 41 |
| **AB611** | SEQ ID NO: 42 | SEQ ID NO: 43 |
| **AB612** | SEQ ID NO: 44 | SEQ ID NO: 45 |
| **AB613** | SEQ ID NO: 46 | SEQ ID NO: 47 |
| **AB614** | SEQ ID NO: 48 | SEQ ID NO: 49 |
| **AB615** | SEQ ID NO: 106 | SEQ ID NO: 49 |
| **AB616** | SEQ ID NO: 107 | SEQ ID NO: 49 |
| **AB617** | SEQ ID NO: 108 | SEQ ID NO: 49 |
| **AB618** | SEQ ID NO: 48 | SEQ ID NO: 109 |
| **AB619** | SEQ ID NO: 48 | SEQ ID NO: 110 |
| **AB620** | SEQ ID NO: 111 | SEQ ID NO: 112 |
| **AB621** | SEQ ID NO: 48 | SEQ ID NO: 113 |
| **AB622** | SEQ ID NO: 48 | SEQ ID NO: 114 |
| **AB623** | SEQ ID NO: 115 | SEQ ID NO: 49 |
| **AB624** | SEQ ID NO: 116 | SEQ ID NO: 49 |
| **AB625** | SEQ ID NO: 117 | SEQ ID NO: 49 |
| **AB626** | SEQ ID NO: 118 | SEQ ID NO: 49 |
| **AB627** | SEQ ID NO: 119 | SEQ ID NO: 49 |

For example, the amino acid sequences of the CDRs included in the variable regions of the humanized antibodies AB610, AB611, AB612, AB613 and AB614 prepared in the present application are shown in Table 4, where were defined by the Kabat and IMGT methods, separately.

**Table 4 CDR sequences of exemplary anti-CD3 humanized antibodies**

| **Antibody No.** | **CDR** | **Kabat** | **IMGT** |
|---|---|---|---|
| **AB610** | CDR-H1 | SEQ ID NO: 29 | SEQ ID NO: 35 |
| | CDR-H2 | SEQ ID NO: 30 | SEQ ID NO: 36 |
| | CDR-H3 | SEQ ID NO: 31 | SEQ ID NO: 37 |
| | CDR-L1 | SEQ ID NO: 50 | SEQ ID NO: 38 |
| | CDR-L2 | SEQ ID NO: 33 | SEQ ID NO: 39 |
| | CDR-L3 | SEQ ID NO: 34 | SEQ ID NO: 34 |
| **AB611** | CDR-H1 | SEQ ID NO: 29 | SEQ ID NO: 35 |
| | CDR-H2 | SEQ ID NO: 30 | SEQ ID NO: 36 |
| | CDR-H3 | SEQ ID NO: 31 | SEQ ID NO: 37 |
| | CDR-L1 | SEQ ID NO: 32 | SEQ ID NO: 38 |
| | CDR-L2 | SEQ ID NO: 33 | SEQ ID NO: 39 |
| | CDR-L3 | SEQ ID NO: 34 | SEQ ID NO: 34 |
| **AB612** | CDR-H1 | SEQ ID NO: 7 | SEQ ID NO: 13 |
| | CDR-H2 | SEQ ID NO: 51 | SEQ ID NO: 55 |
| | CDR-H3 | SEQ ID NO: 52 | SEQ ID NO: 56 |
| | CDR-L1 | SEQ ID NO: 53 | SEQ ID NO: 16 |
| | CDR-L2 | SEQ ID NO: 54 | SEQ ID NO: 17 |
| | CDR-L3 | SEQ ID NO: 12 | SEQ ID NO: 12 |
| **AB613** | CDR-H1 | SEQ ID NO: 7 | SEQ ID NO: 13 |
| | CDR-H2 | SEQ ID NO: 51 | SEQ ID NO: 55 |
| | CDR-H3 | SEQ ID NO: 52 | SEQ ID NO: 56 |
| | CDR-L1 | SEQ ID NO: 10 | SEQ ID NO: 16 |
| | CDR-L2 | SEQ ID NO: 11 | SEQ ID NO: 17 |
| | CDR-L3 | SEQ ID NO: 12 | SEQ ID NO: 12 |
| **AB614** | CDR-H1 | SEQ ID NO: 18 | SEQ ID NO: 24 |
| | CDR-H2 | SEQ ID NO: 57 | SEQ ID NO: 25 |
| | CDR-H3 | SEQ ID NO: 20 | SEQ ID NO: 26 |
| | CDR-L1 | SEQ ID NO: 21 | SEQ ID NO: 27 |
| | CDR-L2 | SEQ ID NO: 58 | SEQ ID NO: 28 |
| | CDR-L3 | SEQ ID NO: 23 | SEQ ID NO: 23 |
| **AB615** | CDR-H1 | SEQ ID NO: 18 | SEQ ID NO: 85 |
| | CDR-H2 | SEQ ID NO: 57 | SEQ ID NO: 25 |
| | CDR-H3 | SEQ ID NO: 20 | SEQ ID NO: 26 |
| | CDR-L1 | SEQ ID NO: 21 | SEQ ID NO: 27 |
| | CDR-L2 | SEQ ID NO: 58 | SEQ ID NO: 28 |
| | CDR-L3 | SEQ ID NO: 23 | SEQ ID NO: 23 |
| **AB616** | CDR-H1 | SEQ ID NO: 18 | SEQ ID NO: 86 |
| | CDR-H2 | SEQ ID NO: 57 | SEQ ID NO: 25 |
| | CDR-H3 | SEQ ID NO: 20 | SEQ ID NO: 26 |
| | CDR-L1 | SEQ ID NO: 21 | SEQ ID NO: 27 |
| | CDR-L2 | SEQ ID NO: 58 | SEQ ID NO: 28 |
| | CDR-L3 | SEQ ID NO: 23 | SEQ ID NO: 23 |
| **AB617** | CDR-H1 | SEQ ID NO: 87 | SEQ ID NO: 88 |
| | CDR-H2 | SEQ ID NO: 57 | SEQ ID NO: 25 |
| | CDR-H3 | SEQ ID NO: 20 | SEQ ID NO: 26 |
| | CDR-L1 | SEQ ID NO: 21 | SEQ ID NO: 27 |
| | CDR-L2 | SEQ ID NO: 58 | SEQ ID NO: 28 |
| | CDR-L3 | SEQ ID NO: 23 | SEQ ID NO: 23 |
| **AB618** | CDR-H1 | SEQ ID NO: 18 | SEQ ID NO: 24 |
| | CDR-H2 | SEQ ID NO: 57 | SEQ ID NO: 25 |
| | CDR-H3 | SEQ ID NO: 20 | SEQ ID NO: 26 |
| | CDR-L1 | SEQ ID NO: 21 | SEQ ID NO: 27 |
| | CDR-L2 | SEQ ID NO: 89 | SEQ ID NO: 90 |
| | CDR-L3 | SEQ ID NO: 23 | SEQ ID NO: 23 |
| **AB619** | CDR-H1 | SEQ ID NO: 18 | SEQ ID NO: 24 |
| | CDR-H2 | SEQ ID NO: 57 | SEQ ID NO: 25 |
| | CDR-H3 | SEQ ID NO: 20 | SEQ ID NO: 26 |
| | CDR-L1 | SEQ ID NO: 21 | SEQ ID NO: 27 |
| | CDR-L2 | SEQ ID NO: 91 | SEQ ID NO: 92 |
| | CDR-L3 | SEQ ID NO: 23 | SEQ ID NO: 23 |
| **AB620** | CDR-H1 | SEQ ID NO: 18 | SEQ ID NO: 86 |
| | CDR-H2 | SEQ ID NO: 57 | SEQ ID NO: 25 |
| | CDR-H3 | SEQ ID NO: 20 | SEQ ID NO: 26 |
| | CDR-L1 | SEQ ID NO: 21 | SEQ ID NO: 27 |
| | CDR-L2 | SEQ ID NO: 91 | SEQ ID NO: 92 |
| | CDR-L3 | SEQ ID NO: 23 | SEQ ID NO: 23 |
| **AB621** | CDR-H1 | SEQ ID NO: 18 | SEQ ID NO: 24 |
| | CDR-H2 | SEQ ID NO: 57 | SEQ ID NO: 25 |
| | CDR-H3 | SEQ ID NO: 20 | SEQ ID NO: 26 |
| | CDR-L1 | SEQ ID NO: 93 | SEQ ID NO: 94 |
| | CDR-L2 | SEQ ID NO: 58 | SEQ ID NO: 28 |
| | CDR-L3 | SEQ ID NO: 23 | SEQ ID NO: 23 |
| **AB622** | CDR-H1 | SEQ ID NO: 18 | SEQ ID NO: 24 |
| | CDR-H2 | SEQ ID NO: 57 | SEQ ID NO: 25 |
| | CDR-H3 | SEQ ID NO: 20 | SEQ ID NO: 26 |
| | CDR-L1 | SEQ ID NO: 21 | SEQ ID NO: 27 |
| | CDR-L2 | SEQ ID NO: 95 | SEQ ID NO: 28 |
| | CDR-L3 | SEQ ID NO: 23 | SEQ ID NO: 23 |
| **AB623** | CDR-H1 | SEQ ID NO: 18 | SEQ ID NO: 24 |
| | CDR-H2 | SEQ ID NO: 57 | SEQ ID NO: 25 |
| | CDR-H3 | SEQ ID NO: 96 | SEQ ID NO: 97 |
| | CDR-L1 | SEQ ID NO: 21 | SEQ ID NO: 27 |
| | CDR-L2 | SEQ ID NO: 58 | SEQ ID NO: 28 |
| | CDR-L3 | SEQ ID NO: 23 | SEQ ID NO: 23 |
| **AB624** | CDR-H1 | SEQ ID NO: 18 | SEQ ID NO: 24 |
| | CDR-H2 | SEQ ID NO: 57 | SEQ ID NO: 25 |
| | CDR-H3 | SEQ ID NO: 98 | SEQ ID NO: 99 |
| | CDR-L1 | SEQ ID NO: 21 | SEQ ID NO: 27 |
| | CDR-L2 | SEQ ID NO: 58 | SEQ ID NO: 28 |
| | CDR-L3 | SEQ ID NO: 23 | SEQ ID NO: 23 |
| **AB625** | CDR-H1 | SEQ ID NO: 18 | SEQ ID NO: 24 |
| | CDR-H2 | SEQ ID NO: 57 | SEQ ID NO: 25 |
| | CDR-H3 | SEQ ID NO: 100 | SEQ ID NO: 101 |
| | CDR-L1 | SEQ ID NO: 21 | SEQ ID NO: 27 |
| | CDR-L2 | SEQ ID NO: 58 | SEQ ID NO: 28 |
| | CDR-L3 | SEQ ID NO: 23 | SEQ ID NO: 23 |
| **AB626** | CDR-H1 | SEQ ID NO: 18 | SEQ ID NO: 24 |
| | CDR-H2 | SEQ ID NO: 57 | SEQ ID NO: 25 |
| | CDR-H3 | SEQ ID NO: 102 | SEQ ID NO: 103 |
| | CDR-L1 | SEQ ID NO: 21 | SEQ ID NO: 27 |
| | CDR-L2 | SEQ ID NO: 58 | SEQ ID NO: 28 |
| | CDR-L3 | SEQ ID NO: 23 | SEQ ID NO: 23 |
| **AB627** | CDR-H1 | SEQ ID NO: 18 | SEQ ID NO: 24 |
| | CDR-H2 | SEQ ID NO: 57 | SEQ ID NO: 25 |
| | CDR-H3 | SEQ ID NO: 104 | SEQ ID NO: 105 |
| | CDR-L1 | SEQ ID NO: 21 | SEQ ID NO: 27 |
| | CDR-L2 | SEQ ID NO: 58 | SEQ ID NO: 28 |
| | CDR-L3 | SEQ ID NO: 23 | SEQ ID NO: 23 |

To obtain a full-length antibody sequence consisting of two heavy chains and two light chains, the sequences of the VHs and the VLs shown in Table 3 were spliced or assembled with a heavy chain constant region (preferably from human IgG1 or IgG4) and a light chain constant region (preferably, from human κ light chain) of an antibody using conventional technologies. For example, in an embodiment, the anti-CD3 antibody molecule includes the heavy chain constant region of human wild-type IgG4 and the human κ light chain constant region shown in Table 5. Alternatively, a modified human IgG4 constant region sequence is used. For example, in an embodiment, as shown in Table 5, the anti-CD3 antibody molecule includes human IgG4 with a mutation at position 228 (for example, from S to P) according to EU numbering. In another embodiment, the anti-CD3 antibody molecule includes the heavy chain constant region of human wild-type IgG1 and the human κ light chain constant region. Alternatively, a modified human IgG1 constant region sequence is used. For example, as shown in Table 5, human IgG1 includes a substitution at position 297 (for example, Asn substituted with Ala) according to EU numbering. In another embodiment, as shown in Table 5, human IgG1 includes a substitution at position 234 according to EU numbering, a substitution at position 235 according to EU numbering, or both the two substitutions (for example, Leu substituted with Ala at position 234 and/or Leu substituted with Ala at position 235).

**Table 5 Amino acid sequences of human IgG heavy chain constant regions and the human κ light chain constant region**

| **Amino acid sequence of the human κ constant region** | |
|---|---|
| SEQ ID NO: 59 | |

| **Amino acid sequence of wild-type IgG1** | |
|---|---|
| SEQ ID NO: 60 | ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKRVEPKSCDKTHTCPPCPAPELLGGPSVFLFPP KPKDTLMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSREEMTKNQVSLTCL VKGFYPSDIAVEWESNGQPENNYKTTPPVLDSDGSFFLYSKL TVDKSRWQQGNVFSCSVMHEALHNHYTQKSLSLSPGK |

| **Amino acid sequence of the constant region of IgG1 (N297A) mutant (EU numbering)** | |
|---|---|
| SEQ ID NO: 61 | ASTKGPSVFPLAPSSKSTSGGTAALGCLVKDYFPEPVTVSWN SGALTSGVHTFPAVLQSSGLYSLSSVVTVPSSSLGTQTYICNV |
| | NHKPSNTKVDKRVEPKSCDKTHTCPPCPAPELLGGPSVFLFPP KPKDTLMISRTPEVTCVVVDVSHEDPEVKFNWYVDGVEVH NAKTKPREEQYASTYRVVSVLTVLHQDWLNGKEYKCKVSN KALPAPIEKTISKAKGQPREPQVYTLPPSREEMTKNQVSLTCL VKGFYPSDIAVEWESNGQPENNYKTTPPVLDSDGSFFLYSKL TVDKSRWQQGNVFSCSVMHEALHNHYTQKSLSLSPGK |

| **Amino acid sequence of the constant region of IgG1 (L234A, L235A) mutant (EU numbering)** | |
|---|---|
| SEQ ID NO: 62 | ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKRVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSREEMTKNQVSLTCL VKGFYPSDIAVEWESNGQPENNYKTTPPVLDSDGSFFLYSKL TVDKSRWQQGNVFSCSVMHEALHNHYTQKSLSLSPGK |

| **Amino acid sequence of wild-type IgG4** | |
|---|---|
| SEQ ID NO: 63 | ASTKGPSVFPLAPCSRSTSESTAALGCLVKDYFPEPVTVSWNS GALTSGVHTFPAVLQSSGLYSLSSVVTVPSSSLGTKTYTCNVD HKPSNTKVDKRVESKYGPPCPSCPAPEFLGGPSVFLFPPKPKD TLMISRTPEVTCVVVDVSQEDPEVQFNWYVDGVEVHNAKT KPREEQFNSTYRVVSVLTVLHQDWLNGKEYKCKVSNKGLPS SIEKTISKAKGQPREPQVYTLPPSQEEMTKNQVSLTCLVKGFY PSDIAVEWESNGQPENNYKTTPPVLDSDGSFFLYSRLTVDKSR WQEGNVFSCSVMHEALHNHYTQKSLSLSLGK |

| **Amino acid sequence of the constant region of IgG4 (S228P) mutant (EU numbering)** | |
|---|---|
| SEQ ID NO: 64 | ASTKGPSVFPLAPCSRSTSESTAALGCLVKDYFPEPVTVSWNS GALTSGVHTFPAVLQSSGLYSLSSVVTVPSSSLGTKTYTCNVD HKPSNTKVDKRVESKYGPPCPPCPAPEFLGGPSVFLFPPKPKD TLMISRTPEVTCVVVDVSQEDPEVQFNWYVDGVEVHNAKT KPREEQFNSTYRVVSVLTVLHQDWLNGKEYKCKVSNKGLPS SIEKTISKAKGQPREPQVYTLPPSQEEMTKNQVSLTCLVKGFY PSDIAVEWESNGQPENNYKTTPPVLDSDGSFFLYSRLTVDKSR WQEGNVFSCSVMHEALHNHYTQKSLSLSLGK |

In an exemplary embodiment, the humanized antibody of the present application includes human IgG4 with a mutation at position 228 (from S to P) according to EU numbering and the human κ light chain constant region. Specifically, the amino acid sequences and the corresponding nucleotide sequences of the heavy and light chains of example humanized antibodies of the present application are shown in Table 6.

**Table 6 Amino acid sequences and the corresponding nucleotide sequences of the heavy and light chains of the humanized antibodies**

| Antibody No. | Heavy chain amino acid sequence | Light chain amino acid sequence | Heavy chain nucleotide sequence | Light chain nucleotide sequence |
|---|---|---|---|---|
| AB610 | SEQ ID NO: 65 | SEQ ID NO: 66 | SEQ ID NO: 67 | SEQ ID NO: 68 |
| AB611 | SEQ ID NO: 69 | SEQ ID NO: 70 | SEQ ID NO: 71 | SEQ ID NO: 72 |
| AB612 | SEQ ID NO: 73 | SEQ ID NO: 74 | SEQ ID NO: 75 | SEQ ID NO: 76 |
| AB613 | SEQ ID NO: 77 | SEQ ID NO: 78 | SEQ ID NO: 79 | SEQ ID NO: 80 |
| AB614 | SEQ ID NO: 81 | SEQ ID NO: 82 | SEQ ID NO: 83 | SEQ ID NO: 84 |

### Example 7 Construction, expression and preparation of an expression vector of an antibody against human CD3

According to the heavy and light chain sequences obtained in the preceding examples, coding cDNA was designed to be inserted into a eukaryotic expression vector pCMAB2M to construct a humanized expression vector. The expression vector plasmid contains cytomegalovirus early gene promoter-enhancer required for high level expression in mammalian cells. Meanwhile, the vector plasmid contains a selectable marker gene to confer ampicillin resistance in bacteria and G418 resistance in mammalian cells. In addition, the vector plasmid contains a dihydrofolate reductase (DHFR) gene, where in a suitable host cell, the antibody gene and the DHFR gene can be co-amplified with methotrexate (MTX).

The constructed recombinant expression vector plasmid was transfected into a mammalian host cell line to express the humanized antibody. To stabilize high level expression, a preferred host cell line is a DHFR-deficient Chinese hamster ovary (CHO) cell (see U.S. Pat. No. 4,818,679). A preferred method of transfection is electroporation, and other methods including calcium phosphate co-precipitation, lipofection and protoplasmic fusion may also be used. During electroporation, 2×10⁷ cells were added, with GenePulser (Bio-Rad Laboratories) set at an electric field of 300 V and a capacitance of 1050 µFd, to a cuvette to be suspended in 0.8 mL of PBS containing 20 µg of the expression vector plasmid. Two days after transfection, 0.2 mg/mL G418 and 200 nM MTX (Sigma) were added. To achieve a relatively high level of expression, the transfected antibody gene was co-amplified with the MTX-inhibited DHFR gene. Transfectants were subcloned by limiting dilution and the secretion rate of each cell line was determined by the ELISA. A cell line expressing the antibody at a high level was selected. The conditioned medium of the antibody was collected for determination of its *in vitro* and *in vivo* biological activity.

### Example 8 Evaluation of in vitro biological functions of CD3 humanized candidate antibodies

### 8.1 Abilities of CD3 humanized antibodies to bind to CD3 antigen

A microtiter plate was coated with 0.1 µg/mL His-labeled human or cynomolgus monkey CD3E (purchased from Aero Biosystems) (100 µL/well) overnight at 4 °C. The coating solution was discarded, and the microtiter plate was blocked with 1% PBSB (200 µL/well), incubated for 1 h at 37 °C and washed with PBS containing 0.05% Tween-20. The diluted CD3 humanized antibodies AB611 and AB614 with a series of concentrations were added (100 µL/well), incubated for 1 h at 37 °C and washed with 0.05% PBST, and HRP-labeled goat anti-human IgG (H+L) (Jackson Laboratory) was added as the detection antibody and incubated for 1 h at 37 °C. The wells were washed three times with 0.05% PBST, added with TMB (100 µL/well) and developed at room temperature for 5 min. 0.2 M H₂SO₄ was added to terminate the reaction, 50 µL/well. Absorption values were read by a microplate reader at double wavelengths 450 nm/620 nm. Plotting was performed by software GraphPad Prism 6 with OD 450 nm/620 nm as the Y-axis and an antibody concentration as the X-axis.

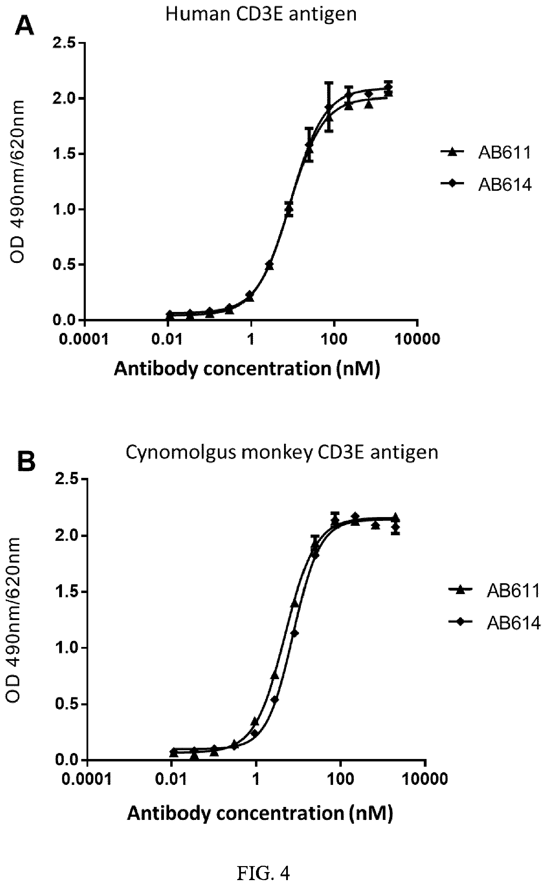

As shown in FIG. 4 and Table 7, at the molecular level, the humanized antibodies AB611 and AB614 can well bind to the human or cynomolgus monkey CD3E antigen, with comparable binding effects.

**Table 7 Determination results of the activities of the CD3 humanized antibodies to bind to the human or cynomolgus monkey CD3**

| Antibody Name | EC₅₀ (pM) | |
|---|---|---|
| | Human CD3E antigen | Cynomolgus monkey CD3E antigen |
| AB611 | 8.334 | 4.835 |
| AB614 | 9.132 | 7.549 |

### 8.2. Activities of the CD3 humanized antibodies to bind to human primary T cells

The human primary T cells were cultured and centrifuged to collect cells, the cells were resuspended in 1% PBSB, the cell density was adjusted to 1×10⁶ cells/mL, and the cells were added to a 96-well U plate (100 µL/well) and blocked for 30 min at 4 °C. The cells were washed once with 1% PBSB. The diluted CD3 humanized antibodies AB611 and AB614 with a series of concentrations were added (100 µL/well) and incubated for 1 h at 4 °C. The wells were centrifuged to remove the supernatant and washed twice with 1% PBSB, and the diluted goat anti-human IgG (H+L) antibody AF647 (Jackson Immuno Research Inc., diluted at 1:250) was added (100 µL/well) and incubated for 1 h at 4 °C in the dark. The wells were centrifuged to remove the supernatant and washed twice with 1% PBSB, and 4% PFA was added (200 µL/well) to resuspend the cells, and signal strength was detected by flow cytometry. With the mean fluorescence intensity as the Y-axis and the antibody concentration as the X-axis, the EC₅₀ values of the CD3 humanized antibodies AB611 and AB614 binding to the human primary T cells were analyzed and calculated by software GraphPad Prism 6.

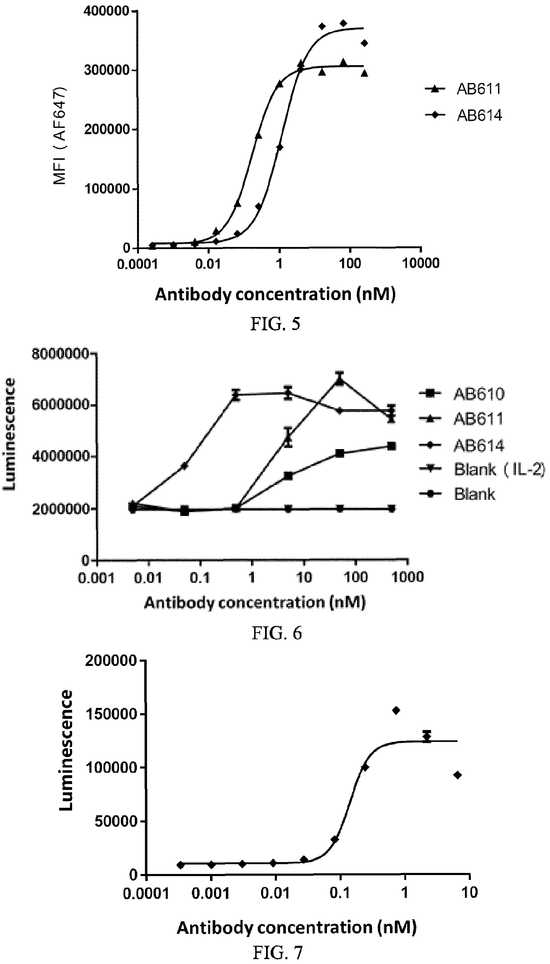

As shown in FIG. 5, at the cellular level, different CD3 humanized antibodies can well bind to the human primary T cells, and the binding signal value of AB611 is lower than that of AB614. The EC₅₀ values of AB611 and AB614 binding to the human primary T cells are 0.174 nM and 1.180 nM, respectively.

### 8.3 Detection of abilities of CD3 humanized antibodies to promote PBMC cell proliferation

The frozen PBMCs were resuscitated, the cell density was adjusted to 5×10⁵ cells/mL, 100 µL/well in a 1640 medium containing 10% FBS, and the cells were added to a 96-well plate. The CD3 humanized antibodies AB610, AB611 and AB614 were diluted to 1 µg/mL in a medium, 10-fold dilution into 6 gradients, 100 µL/well was added to the 96-well plate with two duplicate wells, and they were incubated for 20 h in a 37 °C, 5% CO₂ incubator. 1000 IU/mL IL-2 was added (50 µL/well) to the 96-well plate and cultured for 48 h in the 37 °C, 5% CO₂ incubator. A control group with no antibody and no IL-2 (Blank) and a control group with no antibody and only IL-2 (Blank (IL-2)) were also set. The cells in the 96-well plate were blown uniformly, 100 µL of cell suspension was pipetted into a 96-well white plate, 100 µL of CellTiter-Glo^{®} luminescence cell viability detection reagent (Promega, G7571) was added, and the cells were shaken in a shaker and incubated for 10 min. Cold luminescence values were detected with a microplate reader. Plotting was performed by software GraphPad Prism 6 with the luminescence as the Y-axis and the antibody concentration as the X-axis.

As shown in FIG. 6, the CD3 humanized antibodies AB610, AB611 and AB614 can all promote T cell proliferation, where AB614 has the best effect, followed by AB611 and AB610.

### 8.4 Evaluation of an ability of a CD3 humanized candidate antibody to activate T cells

Jurkat T cells (purchased from BPS Bioscience) containing NFAT RE reporter can overexpress luciferase in the presence of the CD3 monoclonal antibody. The degree of activation of Jurkat T cells was quantified by detecting the activity of luciferase. Specifically, the cell density of NFAT-Jurkat cells was adjusted to 2×10⁶ cells/mL, 50 µL/well. The CD3 humanized antibody AB614 with a series of concentrations was added (50 µL/well) with two duplicate wells and incubated for 4 h in a 37 °C, 5% CO₂ incubator. Steady-Glo^{®} Luciferase (purchased from Promega) was added (100 µL/well) and reacted for 5 min, and then cold luminescence values were detected with a microplate reader. With the luminescence as the Y-axis and the antibody concentration as the X-axis, the EC₅₀ of the CD3 humanized antibody AB614 activating T cells was analyzed and calculated by software GraphPad Prism 6.

As shown in FIG. 7, in the activation assay evaluation, the CD3 humanized antibody AB614 had a good activation effect on T cells with an EC₅₀ of 0.142 nM.

### 8.5 Determination of kinetic constants and affinity of anti-CD3 humanized antibodies through biofilm interferometry

For example, the experimental method for determining the kinetic constants and affinity equilibrium dissociation constants of the humanized antibodies was as described in Example 3. The affinity results of the humanized antibodies are shown in Table 8.

**Table 8 Affinity determination results of the humanized antibodies**

| Antibody | K_{D} (M) | Ka (1/Ms) | Kd (1/s) |
|---|---|---|---|
| AB614 | 1.959E-11 | 7.410E05 | 1.452E-05 |
| AB615 | 6.683E-10 | 2.539E05 | 1.697E-04 |
| AB616 | 1.159E-10 | 2.559E05 | 2.966E-05 |
| AB617 | 2.765E-09 | 2.525E05 | 6.982E-04 |
| AB618 | <1.0E-12 | 5.119E04 | <1.0E-07 |
| AB619 | 1.234E-10 | 2.759E05 | 3.405E-05 |
| AB620 | 1.689E-10 | 2.526E05 | 4.265E-05 |
| AB621 | 2.121E-09 | 1.740E05 | 3.691E-04 |
| AB622 | 1.446E-09 | 2.701E05 | 3.907E-04 |
| AB623 | 2.334E-08 | 5.116E04 | 1.194E-03 |
| AB624 | 6.769E-10 | 2.964E05 | 2.006E-04 |
| AB625 | 1.803E-09 | 1.767E05 | 3.185E-04 |
| AB626 | 1.816E-07 | 9.299E03 | 1.689E-03 |
| AB627 | 2.643E-12 | 7.303E03 | <1.0E-07 |

Though the preferred examples of the present application are illustrated and described, it is to be understood that those skilled in the art may make various changes in accordance with the teachings herein within the scope of the present application.

All the publications mentioned in the present application are incorporated herein by reference as if each publication is separately incorporated herein by reference. In addition, it is to be understood that those skilled in the art, who have read the preceding content of the present application, may make various changes or modifications to the present application, and these equivalent forms also fall within the scope of the appended claims.

## Claims

1. An antibody capable of specifically binding to CD3 or an antigen-binding fragment thereof, wherein the antibody or the antigen-binding fragment thereof comprises a heavy chain variable region (VH) comprising at least one, two or three complementarity-determining regions (CDRs) selected from the group consisting of:
(i) CDR-H1 having a sequence as shown in SEQ ID NO: 7, 13, 18, 24, 29, 35, 85, 86, 87 or 88, or a sequence that has one or more amino acid substitutions, deletions or additions (such as 1, 2 or 3 substitutions, deletions or additions) relative to any of the sequences;
(ii) CDR-H2 having a sequence as shown in SEQ ID NO: 8, 14, 19, 25, 30, 36, 51, 55 or 57, or a sequence that has one or more amino acid substitutions, deletions or additions (such as 1, 2 or 3 substitutions, deletions or additions) relative to any of the sequences; and
(iii) CDR-H3 having a sequence as shown in SEQ ID NO: 9, 15, 20, 26, 31, 37, 52, 56, 96, 97, 98, 99, 100, 101, 102, 103, 104 or 105, or a sequence that has one or more amino acid substitutions, deletions or additions (such as 1, 2 or 3 substitutions, deletions or additions) relative to any of the sequences;
and/or the antibody or the antigen-binding fragment thereof comprises a light chain variable region (VL) comprising at least one, two or three CDRs selected from the group consisting of:
(iv) CDR-L1 having a sequence as shown in SEQ ID NO: 10, 16, 21, 27, 32, 38, 50, 53, 93 or 94, or a sequence that has one or more amino acid substitutions, deletions or additions (such as 1, 2 or 3 substitutions, deletions or additions) relative to any of the sequences;
(v) CDR-L2 having a sequence as shown in SEQ ID NO: 11, 17, 22, 28, 33, 39, 54, 58, 89, 90, 91, 92 or 95, or a sequence that has one or more amino acid substitutions, deletions or additions (such as 1, 2 or 3 substitutions, deletions or additions) relative to any of the sequences; and
(vi) CDR-L3 having a sequence as shown in SEQ ID NO: 12, 23 or 34, or a sequence that has one or more amino acid substitutions, deletions or additions (such as 1, 2 or 3 substitutions, deletions or additions) relative to any of the sequences;
preferably, the substitutions in any one of (i) to (vi) are conservative substitutions.

2. The antibody or the antigen-binding fragment thereof according to claim 1, wherein the antibody or the antigen-binding fragment thereof comprises three VH CDRs and three VL CDRs selected from the group consisting of:
(1) CDR-H1, CDR-H2, CDR-H3, CDR-L1, CDR-L2 and CDR-L3 having sequences as shown in SEQ ID NOs: 7, 8, 9, 10, 11 and 12 respectively or sequences that have one or more amino acid substitutions, deletions or additions (such as 1, 2 or 3 substitutions, deletions or additions) relative to any of the sequences;
(2) CDR-H1, CDR-H2, CDR-H3, CDR-L1, CDR-L2 and CDR-L3 having sequences as shown in SEQ ID NOs: 7, 51, 52, 53, 54 and 12 respectively or sequences that have one or more amino acid substitutions, deletions or additions (such as 1, 2 or 3 substitutions, deletions or additions) relative to any of the sequences;
(3) CDR-H1, CDR-H2, CDR-H3, CDR-L1, CDR-L2 and CDR-L3 having sequences as shown in SEQ ID NOs: 7, 51, 52, 10, 11 and 12 respectively or sequences that have one or more amino acid substitutions, deletions or additions (such as 1, 2 or 3 substitutions, deletions or additions) relative to any of the sequences;
(4) CDR-H1, CDR-H2, CDR-H3, CDR-L1, CDR-L2 and CDR-L3 having sequences as shown in SEQ ID NOs: 13, 14, 15, 16, 17 and 12 respectively or sequences that have one or more amino acid substitutions, deletions or additions (such as 1, 2 or 3 substitutions, deletions or additions) relative to any of the sequences;
(5) CDR-H1, CDR-H2, CDR-H3, CDR-L1, CDR-L2 and CDR-L3 having sequences as shown in SEQ ID NOs: 13, 55, 56, 16, 17 and 12 respectively or sequences that have one or more amino acid substitutions, deletions or additions (such as 1, 2 or 3 substitutions, deletions or additions) relative to any of the sequences;
(6) CDR-H1, CDR-H2, CDR-H3, CDR-L1, CDR-L2 and CDR-L3 having sequences as shown in SEQ ID NOs: 18, 19, 20, 21, 22 and 23 respectively or sequences that have one or more amino acid substitutions, deletions or additions (such as 1, 2 or 3 substitutions, deletions or additions) relative to any of the sequences;
(7) CDR-H1, CDR-H2, CDR-H3, CDR-L1, CDR-L2 and CDR-L3 having sequences as shown in SEQ ID NOs: 18, 57, 20, 21, 58 and 23 respectively or sequences that have one or more amino acid substitutions, deletions or additions (such as 1, 2 or 3 substitutions, deletions or additions) relative to any of the sequences;
(8) CDR-H1, CDR-H2, CDR-H3, CDR-L1, CDR-L2 and CDR-L3 having sequences as shown in SEQ ID NOs: 24, 25, 26, 27, 28 and 23 respectively or sequences that have one or more amino acid substitutions, deletions or additions (such as 1, 2 or 3 substitutions, deletions or additions) relative to any of the sequences;
(9) CDR-H1, CDR-H2, CDR-H3, CDR-L1, CDR-L2 and CDR-L3 having sequences as shown in SEQ ID NOs: 29, 30, 31, 32, 33 and 34 respectively or sequences that have one or more amino acid substitutions, deletions or additions (such as 1, 2 or 3 substitutions, deletions or additions) relative to any of the sequences;
(10) CDR-H1, CDR-H2, CDR-H3, CDR-L1, CDR-L2 and CDR-L3 having sequences as shown in SEQ ID NOs: 29, 30, 31, 50, 33 and 34 respectively or sequences that have one or more amino acid substitutions, deletions or additions (such as 1, 2 or 3 substitutions, deletions or additions) relative to any of the sequences;
(11) CDR-H1, CDR-H2, CDR-H3, CDR-L1, CDR-L2 and CDR-L3 having sequences as shown in SEQ ID NOs: 35, 36, 37, 38, 39 and 34 respectively or sequences that have one or more amino acid substitutions, deletions or additions (such as 1, 2 or 3 substitutions, deletions or additions) relative to any of the sequences;
(12) CDR-H1, CDR-H2, CDR-H3, CDR-L1, CDR-L2 and CDR-L3 having sequences as shown in SEQ ID NOs: 87, 57, 20, 21, 58 and 23 respectively or sequences that have one or more amino acid substitutions, deletions or additions (such as 1, 2 or 3 substitutions, deletions or additions) relative to any of the sequences;
(13) CDR-H1, CDR-H2, CDR-H3, CDR-L1, CDR-L2 and CDR-L3 having sequences as shown in SEQ ID NOs: 18, 57, 20, 21, 89 and 23 respectively or sequences that have one or more amino acid substitutions, deletions or additions (such as 1, 2 or 3 substitutions, deletions or additions) relative to any of the sequences;
(14) CDR-H1, CDR-H2, CDR-H3, CDR-L1, CDR-L2 and CDR-L3 having sequences as shown in SEQ ID NOs: 18, 57, 20, 21, 91 and 23 respectively or sequences that have one or more amino acid substitutions, deletions or additions (such as 1, 2 or 3 substitutions, deletions or additions) relative to any of the sequences;
(15) CDR-H1, CDR-H2, CDR-H3, CDR-L1, CDR-L2 and CDR-L3 having sequences as shown in SEQ ID NOs: 18, 57, 20, 93, 58 and 23 respectively or sequences that have one or more amino acid substitutions, deletions or additions (such as 1, 2 or 3 substitutions, deletions or additions) relative to any of the sequences;
(16) CDR-H1, CDR-H2, CDR-H3, CDR-L1, CDR-L2 and CDR-L3 having sequences as shown in SEQ ID NOs: 18, 57, 20, 21, 95 and 23 respectively or sequences that have one or more amino acid substitutions, deletions or additions (such as 1, 2 or 3 substitutions, deletions or additions) relative to any of the sequences;
(17) CDR-H1, CDR-H2, CDR-H3, CDR-L1, CDR-L2 and CDR-L3 having sequences as shown in SEQ ID NOs: 18, 57, 96, 21, 58 and 23 respectively or sequences that have one or more amino acid substitutions, deletions or additions (such as 1, 2 or 3 substitutions, deletions or additions) relative to any of the sequences;
(18) CDR-H1, CDR-H2, CDR-H3, CDR-L1, CDR-L2 and CDR-L3 having sequences as shown in SEQ ID NOs: 18, 57, 98, 21, 58 and 23 respectively or sequences that have one or more amino acid substitutions, deletions or additions (such as 1, 2 or 3 substitutions, deletions or additions) relative to any of the sequences;
(19) CDR-H1, CDR-H2, CDR-H3, CDR-L1, CDR-L2 and CDR-L3 having sequences as shown in SEQ ID NOs: 18, 57, 100, 21, 58 and 23 respectively or sequences that have one or more amino acid substitutions, deletions or additions (such as 1, 2 or 3 substitutions, deletions or additions) relative to any of the sequences;
(20) CDR-H1, CDR-H2, CDR-H3, CDR-L1, CDR-L2 and CDR-L3 having sequences as shown in SEQ ID NOs: 18, 57, 102, 21, 58 and 23 respectively or sequences that have one or more amino acid substitutions, deletions or additions (such as 1, 2 or 3 substitutions, deletions or additions) relative to any of the sequences;
(21) CDR-H1, CDR-H2, CDR-H3, CDR-L1, CDR-L2 and CDR-L3 having sequences as shown in SEQ ID NOs: 18, 57, 104, 21, 58 and 23 respectively or sequences that have one or more amino acid substitutions, deletions or additions (such as 1, 2 or 3 substitutions, deletions or additions) relative to any of the sequences;
(22) CDR-H1, CDR-H2, CDR-H3, CDR-L1, CDR-L2 and CDR-L3 having sequences as shown in SEQ ID NOs: 85, 25, 26, 27, 28 and 23 respectively or sequences that have one or more amino acid substitutions, deletions or additions (such as 1, 2 or 3 substitutions, deletions or additions) relative to any of the sequences;
(23) CDR-H1, CDR-H2, CDR-H3, CDR-L1, CDR-L2 and CDR-L3 having sequences as shown in SEQ ID NOs: 86, 25, 26, 27, 28 and 23 respectively or sequences that have one or more amino acid substitutions, deletions or additions (such as 1, 2 or 3 substitutions, deletions or additions) relative to any of the sequences;
(24) CDR-H1, CDR-H2, CDR-H3, CDR-L1, CDR-L2 and CDR-L3 having sequences as shown in SEQ ID NOs: 88, 25, 26, 27, 28 and 23 respectively or sequences that have one or more amino acid substitutions, deletions or additions (such as 1, 2 or 3 substitutions, deletions or additions) relative to any of the sequences;
(25) CDR-H1, CDR-H2, CDR-H3, CDR-L1, CDR-L2 and CDR-L3 having sequences as shown in SEQ ID NOs: 24, 25, 26, 27, 90 and 23 respectively or sequences that have one or more amino acid substitutions, deletions or additions (such as 1, 2 or 3 substitutions, deletions or additions) relative to any of the sequences;
(26) CDR-H1, CDR-H2, CDR-H3, CDR-L1, CDR-L2 and CDR-L3 having sequences as shown in SEQ ID NOs: 24, 25, 26, 27, 92 and 23 respectively or sequences that have one or more amino acid substitutions, deletions or additions (such as 1, 2 or 3 substitutions, deletions or additions) relative to any of the sequences;
(27) CDR-H1, CDR-H2, CDR-H3, CDR-L1, CDR-L2 and CDR-L3 having sequences as shown in SEQ ID NOs: 86, 25, 26, 27, 92 and 23 respectively or sequences that have one or more amino acid substitutions, deletions or additions (such as 1, 2 or 3 substitutions, deletions or additions) relative to any of the sequences;
(28) CDR-H1, CDR-H2, CDR-H3, CDR-L1, CDR-L2 and CDR-L3 having sequences as shown in SEQ ID NOs: 24, 25, 26, 94, 28 and 23 respectively or sequences that have one or more amino acid substitutions, deletions or additions (such as 1, 2 or 3 substitutions, deletions or additions) relative to any of the sequences;
(29) CDR-H1, CDR-H2, CDR-H3, CDR-L1, CDR-L2 and CDR-L3 having sequences as shown in SEQ ID NOs: 24, 25, 97, 27, 28 and 23 respectively or sequences that have one or more amino acid substitutions, deletions or additions (such as 1, 2 or 3 substitutions, deletions or additions) relative to any of the sequences;
(30) CDR-H1, CDR-H2, CDR-H3, CDR-L1, CDR-L2 and CDR-L3 having sequences as shown in SEQ ID NOs: 24, 25, 99, 27, 28 and 23 respectively or sequences that have one or more amino acid substitutions, deletions or additions (such as 1, 2 or 3 substitutions, deletions or additions) relative to any of the sequences;
(31) CDR-H1, CDR-H2, CDR-H3, CDR-L1, CDR-L2 and CDR-L3 having sequences as shown in SEQ ID NOs: 24, 25, 101, 27, 28 and 23 respectively or sequences that have one or more amino acid substitutions, deletions or additions (such as 1, 2 or 3 substitutions, deletions or additions) relative to any of the sequences;
(32) CDR-H1, CDR-H2, CDR-H3, CDR-L1, CDR-L2 and CDR-L3 having sequences as shown in SEQ ID NOs: 24, 25, 103, 27, 28 and 23 respectively or sequences that have one or more amino acid substitutions, deletions or additions (such as 1, 2 or 3 substitutions, deletions or additions) relative to any of the sequences; and
(33) CDR-H1, CDR-H2, CDR-H3, CDR-L1, CDR-L2 and CDR-L3 having sequences as shown in SEQ ID NOs: 24, 25, 105, 27, 28 and 23 respectively or sequences that have one or more amino acid substitutions, deletions or additions (such as 1, 2 or 3 substitutions, deletions or additions) relative to any of the sequences.

3. The antibody or the antigen-binding fragment thereof according to claim 2, wherein the antibody or the antigen-binding fragment thereof is murine or chimeric, and the heavy chain variable region thereof comprises a heavy chain FR region of murine IgG1, IgG2 or IgG3 or a variant thereof; and the light chain variable region thereof comprises a light chain FR region of a murine κ or λ chain or a variant thereof.

4. The antibody or the antigen-binding fragment thereof according to claim 3, wherein the antibody or the antigen-binding fragment thereof comprises VH and VL sequences selected from the group consisting of:
(i) a VH domain comprising an amino acid sequence as shown in SEQ ID NO: 1 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 1; and a VL domain comprising an amino acid sequence as shown in SEQ ID NO: 2 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 2;
(ii) a VH domain comprising an amino acid sequence as shown in SEQ ID NO: 3 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 3; and a VL domain comprising an amino acid sequence as shown in SEQ ID NO: 4 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 4; and
(iii) a VH domain comprising an amino acid sequence as shown in SEQ ID NO: 5 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 5; and a VL domain comprising an amino acid sequence as shown in SEQ ID NO: 6 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 6.

5. The antibody or the antigen-binding fragment thereof according to claim 2, wherein the antibody or the antigen-binding fragment thereof is humanized.

6. The antibody or the antigen-binding fragment thereof according to claim 5, wherein the antibody or the antigen-binding fragment thereof comprises VH and VL sequences selected from the group consisting of:
(1) a VH domain comprising an amino acid sequence as shown in SEQ ID NO: 40 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 40; and a VL domain comprising an amino acid sequence as shown in SEQ ID NO: 41 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 41;
(2) a VH domain comprising an amino acid sequence as shown in SEQ ID NO: 42 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 42; and a VL domain comprising an amino acid sequence as shown in SEQ ID NO: 43 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 43;
(3) a VH domain comprising an amino acid sequence as shown in SEQ ID NO: 44 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 44; and a VL domain comprising an amino acid sequence as shown in SEQ ID NO: 45 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 45;
(4) a VH domain comprising an amino acid sequence as shown in SEQ ID NO: 46 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 46; and a VL domain comprising an amino acid sequence as shown in SEQ ID NO: 47 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 47;
(5) a VH domain comprising an amino acid sequence as shown in SEQ ID NO: 48 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 48; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(6) a VH domain comprising an amino acid sequence as shown in SEQ ID NO: 106 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 106; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(7) a VH domain comprising an amino acid sequence as shown in SEQ ID NO: 107 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 107; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(8) a VH domain comprising an amino acid sequence as shown in SEQ ID NO: 108 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 108; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(9) a VH domain comprising an amino acid sequence as shown in SEQ ID NO: 48 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 48; and a VL domain comprising an amino acid sequence as shown in SEQ ID NO: 109 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 109;
(10) a VH domain comprising an amino acid sequence as shown in SEQ ID NO: 48 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 48; and a VL domain comprising an amino acid sequence as shown in SEQ ID NO: 110 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 110;
(11) a VH domain comprising an amino acid sequence as shown in SEQ ID NO: 111 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 111; and a VL domain comprising an amino acid sequence as shown in SEQ ID NO: 112 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 112;
(12) a VH domain comprising an amino acid sequence as shown in SEQ ID NO: 48 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 48; and a VL domain comprising an amino acid sequence as shown in SEQ ID NO: 113 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 113;
(13) a VH domain comprising an amino acid sequence as shown in SEQ ID NO: 48 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 48; and a VL domain comprising an amino acid sequence as shown in SEQ ID NO: 114 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 114;
(14) a VH domain comprising an amino acid sequence as shown in SEQ ID NO: 115 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 115; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(15) a VH domain comprising an amino acid sequence as shown in SEQ ID NO: 116 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 116; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(16) a VH domain comprising an amino acid sequence as shown in SEQ ID NO: 117 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 117; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49;
(17) a VH domain comprising an amino acid sequence as shown in SEQ ID NO: 118 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 118; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49; and
(18) a VH domain comprising an amino acid sequence as shown in SEQ ID NO: 119 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 119; and a VL domain comprising an amino acid sequence as shown in SEQ ID NO: 49 or having a sequence that is substantially identical to (for example, has at least 80%, 85%, 90%, 92%, 95%, 97%, 98%, 99% or higher identity to or has one or more amino acid substitutions (such as conservative substitutions) relative to) SEQ ID NO: 49.

7. The antibody according to claim 6, wherein the antibody comprises a heavy chain constant region and a light chain constant region derived from human immunoglobulins; preferably, the heavy chain constant region is selected from heavy chain constant regions of human IgG1, IgG2, IgG3, IgG4, IgM, IgA1, IgA2, IgD and IgE; more preferably, the heavy chain constant region is selected from heavy chain constant regions of human IgG1, IgG2, IgG3 and IgG4; the heavy chain constant region has a natural sequence or a sequence having one or more amino acid substitutions, deletions or additions relative to a natural sequence from which the heavy chain constant region is derived; and the light chain constant region is preferably a constant region of a human κappa chain as shown in SEQ ID NO: 59.

8. The antibody according to claim 7, wherein the antibody comprises a heavy chain constant region selected from the group consisting of:
(i) a heavy chain constant region of wild-type human IgG1, as shown in SEQ ID NO: 60;
(ii) a heavy chain constant region of human IgG1 containing an Asn297Ala mutation, as shown in SEQ ID NO: 61;
(iii) a heavy chain constant region of human IgG1 containing Leu234Ala and Leu235Ala mutations, as shown in SEQ ID NO: 62;
(iv) a heavy chain constant region of wild-type human IgG4, as shown in SEQ ID NO: 63; and
(v) a heavy chain constant region of human IgG4 containing an S228P mutation, as shown in SEQ ID NO: 64.

9. The antibody according to claim 7 or 8, wherein the antibody comprises a full-length amino acid sequence selected from the group consisting of:
(i) a heavy chain having an amino acid sequence as shown in SEQ ID NO: 65 and a light chain having an amino acid sequence as shown in SEQ ID NO: 66; sequences that have one or more substitutions, deletions or additions (such as 1, 2, 3, 4 or 5 substitutions, deletions or additions) relative to any of the sequences; or sequences that have at least 80%, at least 85%, at least 90%, at least 91%, at least 92%, at least 93%, at least 94%, at least 95%, at least 96%, at least 97%, at least 98%, at least 99% or higher identity to any of the sequences;
(ii) a heavy chain having an amino acid sequence as shown in SEQ ID NO: 69 and a light chain having an amino acid sequence as shown in SEQ ID NO: 70; sequences that have one or more substitutions, deletions or additions (such as 1, 2, 3, 4 or 5 substitutions, deletions or additions) relative to any of the sequences; or sequences that have at least 80%, at least 85%, at least 90%, at least 91%, at least 92%, at least 93%, at least 94%, at least 95%, at least 96%, at least 97%, at least 98%, at least 99% or higher identity to any of the sequences;
(iii) a heavy chain having an amino acid sequence as shown in SEQ ID NO: 73 and a light chain having an amino acid sequence as shown in SEQ ID NO: 74; sequences that have one or more substitutions, deletions or additions (such as 1, 2, 3, 4 or 5 substitutions, deletions or additions) relative to any of the sequences; or sequences that have at least 80%, at least 85%, at least 90%, at least 91%, at least 92%, at least 93%, at least 94%, at least 95%, at least 96%, at least 97%, at least 98%, at least 99% or higher identity to any of the sequences;
(iv) a heavy chain having an amino acid sequence as shown in SEQ ID NO: 77 and a light chain having an amino acid sequence as shown in SEQ ID NO: 78; sequences that have one or more substitutions, deletions or additions (such as 1, 2, 3, 4 or 5 substitutions, deletions or additions) relative to any of the sequences; or sequences that have at least 80%, at least 85%, at least 90%, at least 91%, at least 92%, at least 93%, at least 94%, at least 95%, at least 96%, at least 97%, at least 98%, at least 99% or higher identity to any of the sequences; and
(v) a heavy chain having an amino acid sequence as shown in SEQ ID NO: 81 and a light chain having an amino acid sequence as shown in SEQ ID NO: 82; sequences that have one or more substitutions, deletions or additions (such as 1, 2, 3, 4 or 5 substitutions, deletions or additions) relative to any of the sequences; or sequences that have at least 80%, at least 85%, at least 90%, at least 91%, at least 92%, at least 93%, at least 94%, at least 95%, at least 96%, at least 97%, at least 98%, at least 99% or higher identity to any of the sequences.

10. A DNA molecule encoding the antibody or the antigen-binding fragment thereof according to any one of claims 1 to 9.

11. The DNA molecule according to claim 10, wherein a DNA molecule encoding a heavy chain of the antibody has a nucleotide sequence as shown in SEQ ID NO: 67, 71, 75, 79 or 83, and a DNA molecule encoding a light chain of the antibody has a nucleotide sequence as shown in SEQ ID NO: 68, 72, 76, 80 or 84.

12. A vector, comprising the DNA molecule according to claim 10 or 11.

13. A host cell, comprising the vector according to claim 12, wherein the host cell comprises a prokaryotic cell, a yeast cell or a mammalian cell.

14. A pharmaceutical composition, comprising the antibody or the antigen-binding fragment thereof according to any one of claims 1 to 9 and a pharmaceutically acceptable excipient, carrier or diluent.

15. A method for preparing the antibody or the antigen-binding fragment thereof according to any one of claims 1 to 9, comprising: (a) obtaining genes of the antibody or the antigen-binding fragment thereof, and constructing an expression vector of the antibody or the antigen-binding fragment thereof; (b) transfecting the expression vector into a host cell by a genetic engineering method; (c) culturing the host cell under a condition that allows the antibody or the antigen-binding fragment thereof to be produced; and (d) separating and purifying the produced antibody or antigen-binding fragment thereof;
wherein the expression vector in step (a) is one or more selected from a plasmid, a bacterium and a virus; preferably, the expression vector is a pcDNA3.1 vector;
wherein the host cell into which the constructed vector is transfected by the genetic engineering method in step (b) comprises a prokaryotic cell, a yeast cell or a mammalian cell, such as a CHO cell, an NS0 cell or another mammalian cell; and
wherein the antibody or the antigen-binding fragment thereof is separated and purified in step (d) by a conventional immunoglobulin purification method comprising protein A affinity chromatography and ion exchange, hydrophobic chromatography or molecular sieve.

16. Use of the antibody or the antigen-binding fragment thereof according to any one of claims 1 to 9 for preparing a medicament, wherein the medicament is used for preparing a medication or formulation for prevention and/or treatment of a tumor, an organ transplantation rejection or an autoimmune disease.

17. The use according to claim 16, wherein the tumor is selected from a solid tumor, a hematological tumor (such as leukemia, lymphoma and myeloma such as multiple myeloma) and a metastatic disease; for example, the tumor comprises, but is not limited to, lung cancer, melanoma, renal cancer, liver cancer, myeloma, breast cancer, colorectal cancer, leukemia or a metastatic disease of cancer; the organ transplantation rejection is selected from abnormal immune responses associated with a cell, a tissue or organ transplantation such as kidney, liver and heart transplantation, comprising, but not limited to, a graft-versus-host disease and an allograft rejection; and the autoimmune disease is selected from rheumatoid arthritis, juvenile rheumatoid arthritis, psoriasis, systemic lupus erythematosus, primary biliary cirrhosis, autoimmune hemolytic anemia, aplastic anemia, autoimmune thrombocytopenic purpura, idiopathic thrombocytopenic purpura, diabetes, encephalomyelitis, Graves' disease, myasthenia gravis, Wegener's disease, autoimmune hepatitis and multiple sclerosis.

18. A bispecific molecule comprising the antibody or the antigen-binding fragment thereof according to any one of claims 1 to 9, preferably, further comprising, but not limited to, an antibody against a molecule selected from the group consisting of CD19, CD20, CD22, CD25, CD30, CD33, CD38, CD39, CD40, CD47, CD52, CD73, CD74, CD123, CD133, CD138, BCMA, CA125, CEA, CS1, DLL3, DLL4, EGFR, EpCAM, FLT3, gpA33, GPC-3, Her2, MEGE-A3, NYESO1, PSMA, TAG-72, CIX, folate-binding protein, GD2, GD3, GM2, VEGF, VEGFR2, VEGFR3, cadherin, integrin, mesothelin, Claudin18, αVβ3, α5β1, ERBB3, c-MET, IGF1R, EPHA3, TRAILR1, TRAILR2, RANKL, a B7 protein family, a mucin family, FAP and tenascin.

19. A method for preventing and/or treating a tumor, an organ transplantation rejection or an autoimmune disease in a subject (such as a human), comprising administering an effective amount of the antibody or the antigen-binding fragment thereof according to any one of claims 1 to 9 or the pharmaceutical composition according to claim 14 to the subject in need.

20. A diagnostic or therapeutic kit, comprising the antibody or the antigen-binding fragment thereof according to any one of claims 1 to 9 and instructions for use.
